(19) Europäisches Patentamt
European Patent Office
Office européen des brevets

(11) Numéro de publication : **0 480 829 B1**

# (12) FASCICULE DE BREVET EUROPEEN

(45) Date de publication du fascicule du brevet : **06.04.94 Bulletin 94/14**

(51) Int. Cl.$^5$ : **A61K 7/13**, C09B 1/28

(21) Numéro de dépôt : **91402694.3**

(22) Date de dépôt : **09.10.91**

## (54) Composition de teinture des cheveux contenant des anthraquinones.

(30) Priorité : **12.10.90 FR 9012628**

(43) Date de publication de la demande : **15.04.92 Bulletin 92/16**

(45) Mention de la délivrance du brevet : **06.04.94 Bulletin 94/14**

(84) Etats contractants désignés : **AT BE CH DE DK ES GB GR IT LI NL SE**

(56) Documents cités :
**DE-A- 1 469 797**
**GB-A- 489 697**
**GB-A- 2 093 867**
**US-A- 2 199 813**
**US-A- 3 806 525**

(73) Titulaire : **L'OREAL**
**14, Rue Royale**
**F-75008 Paris (FR)**

(72) Inventeur : **Junino, Alex**
**16, rue Docteur Bergonié**
**F-93180 Livry-Gargan (FR)**
Inventeur : **Genet, Alain**
**9, rue des Coquelicots**
**F-93600 Aulnay-sous-Bois (FR)**

(74) Mandataire : **Casalonga, Axel et al**
**BUREAU D.A. CASALONGA - JOSSE**
**Morassistrasse 8**
**D-80469 München (DE)**

EP 0 480 829 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Utilisation de 1,4-di(mono ou poly)hydroxyalkylamino-9,10-anthraquinones pour la teinture de fibres kératiniques humaines, composition cosmétique les contenant en association avec des colorants azoïques et nitrés benzéniques, et procédé de teinture des cheveux les mettant en oeuvre.

La présente invention concerne l'utilisation de 1,4-di(mono ou poly)hydroxyalkylamino-9,10-anthraquinones pour la teinture des fibres kératiniques humaines, les compositions cosmétiques tinctoriales contenant de telles anthraquinones, les compositions tinctoriales de fibres kératiniques contenant de telles anthraquinones en association avec des colorants nitrés du type benzénique, des colorants azoïques, pour la coloration directe et/ou avec des colorants d'oxydation pour la coloration d'oxydation ainsi que les procédés de teinture des fibres kératiniques par coloration directe ou par coloration d'oxydation.

Comme colorants pour la teinture des fibres kératiniques humaines, il est connu d'utiliser, soit des colorants d'oxydation, soit des colorants directs.

Les colorants d'oxydation conduisent à des nuances couvrantes et tenaces, mais leur très grande ténacité et leur très grande affinité pour le cheveu, impliquent le plus souvent l'apparition du phénomène connu dans l'art de la coloration, à savoir le phénomène de démarcation entre les pointes et demi-longueurs teintes, d'une part, et les racines non teintes, d'autre part.

Dans le cas des colorants directs et en particulier des colorants nitrés du type benzénique, ce défaut n'apparaît pas et c'est pourquoi les colorants nitrés du type benzénique sont souvent utilisés dans le domaine de la coloration directe. Cependant, ces colorants directs présentent le désavantage, dans certains cas, d'avoir une affinité insuffisante pour les cheveux naturels non sensibilisés.

Pour pallier à ce défaut, on a tenté d'associer aux colorants nitrés du type benzénique, certains colorants et notamment des colorants cosmétiques du type azoïque ou anthraquinoniques aminés.

Dans la classe des colorants anthraquinoniques aminés, on a décrit notamment le 1,4,5,8-tétraaminoanthraquinone (color index 64500) qui présente le désavantage d'être très peu soluble dans les solvants organiques ou dans les milieux eau/solvants constituant la base des produits de coloration capillaire.

La demanderesse a maintenant mis en évidence, de façon surprenante, que certaines di-(mono ou poly)hydroxyalkylamino-9,10-anthraquinones présentent non seulement l'avantage d'une excellente solubilité dans les milieux solvants et les supports de teintures usuels, mais encore celui de donner des teintures tenaces stables, notamment aux lavages, à la lumière, aux agressions atmosphériques, aux agents chimiques, à la transpiration, et enfin celui d'une bonne innocuité qui rend possible leur utilisation en teintures des fibres kératiniques humaines.

C'est pourquoi la présente invention concerne l'utilisation, pour la teinture des fibres kératiniques humaines, des anthraquinones de formule :

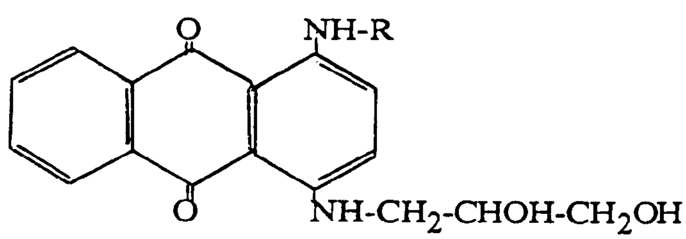

(I)

où R représente un radical monohydroxyalkyle en $C_2$ à $C_6$, ou un radical polyhydroxyalkyle en $C_3$ à $C_6$, pour la teinture des fibres kératiniques humaines.

Dans la formule (I), on entend par alkyle en $C_3$ à $C_6$, les radicaux alkyle linéaires ou ramifiés, comportant de 3 à 6 atomes de carbone. Parmi les polyhydroxyalkyles, sont préférés les dihydroxyalkyles.

Ces anthraquinones de formule (I) sont connues par le brevet US-A-2.199.813. Ce brevet ne décrit cependant ces composés que comme colorants de fibres synthétiques.

L'invention concerne donc en particulier l'utilisation de la 1,4-di-(β, γ-dihydroxypropylamino)9,10-anthraquinone (formule (I) où R représente $CH_2$-CHOH-$CH_2$OH)) et de la 1-(β-hydroxyéthylamino)4-(β, γ-dihydroxypropylamino)9,10-anthraquinone (formule (I) où R représente $CH_2$-$CH_2$OH) pour la teinture des fibres kératiniques humaines.

Un autre objet de l'invention est constitué par les compositions cosmétiques comportant une ou plusieurs anthraquinones de formule :

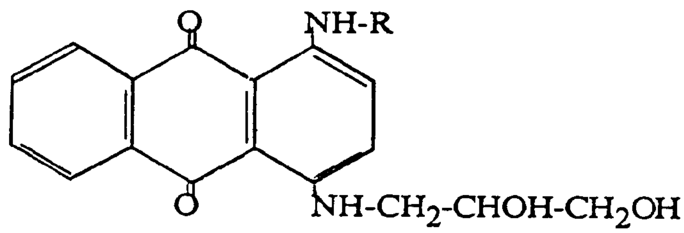

(I)

et en outre l'un au moins des adjuvants cosmétiques choisis parmi les agents tensio-actifs anioniques, cationiques, non-ioniques, amphotères ou leurs mélanges, les épaississants, les agents dispersants, les agents de pénétration, les séquestrants, les agents filmogènes, les agents de traitement des cheveux, les tampons, les parfums, les agents alcalinisants ou acidifiants, les agents opacifiants ou les conservateurs.

Parmi ces compositions cosmétiques, on préfère celles qui contiennent les anthraquinones de formule (I) où R représente $CH_2$-CHOH-$CH_2$OH ou $CH_2$-$CH_2$OH.

Un autre objet de l'invention est constitué par les compositions tinctoriales des fibres kératiniques, caractérisé en ce qu'elles comportent une ou plusieurs anthraquinones de formule (I) :

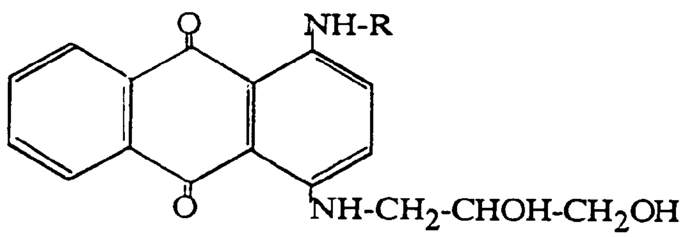

(I)

où R a la signification indiquée ci-dessus, en association avec un ou plusieurs autres colorants choisis parmi :

(i) les colorants nitrés du type benzénique;

(ii) les colorants du type azoïque;

(iii) les précurseurs de colorants d'oxydation.

Selon un premier mode de réalisation de l'invention, le ou les colorants anthraquinoniques de formule (I) est/sont utilisé(s) en association avec au moins un colorant nitré du type benzénique.

Parmi les colorants nitrés du type benzénique, sont préférés les colorants nitrés benzéniques bleus ou violets appartenant à la classe des nitroparaphénylènediamines, ayant une nuance ou "hue" selon Munsell, comprise entre 2,5 B et 10 RP, selon la publication de Officiel Digest, Avril 1975, page 375, figure 2.

On peut cependant également utiliser, en association avec la ou les aminoanthraquinones de formule (I), d'autres colorants nitrobenzéniques donnant des nuances non comprises entre 2,5 B et 10 RP, jaunes, oranges ou rouges, appartenant à la série des nitrophénylènediamines ou à d'autres séries de colorants nitrobenzéniques, et notamment par exemple des nitroaminophénols, des nitroaminoalcoxybenzènes ou des nitroaminohydroxyalcoxybenzènes.

Parmi les colorants nitrés du type benzénique, bleus ou violets préférés, appartenant à la classe des nitroparaphénylènediamines, on peut citer :

- le 2-N-méthylamino 5-[N,N-bis(β-hydroxyéthyl)amino]nitrobenzène;
- le 2-N-méthylamino 5-[N-méthyl,N-(β-hydroxyéthyl)amino,]nitrobenzène;
- le 2-(N-β-hydroxyéthyl)amino 5-[N,N-bis(β-hydroxyéthyl)amino] nitrobenzène;
- le 2-(N-β-hydroxyéthyl)amino 5-[N-méthyl,N-(β-hydroxyéthyl)amino] nitrobenzène;
- le 2-(N-γ-hydroxypropylamino) 5-/N,N-bis(β-hydroxyéthyl)amino] nitrobenzène;
- le 2-(N-β-aminoéthylamino) 5-[N,N-bis(β-hydroxyéthyl)amino]nitrobenzène;
- le 2-N-méthylamino 5-[N-méthyl,N-(β,γ-dihydroxypropyl)amino] nitrobenzène;
- le 2-amino 5-[N,N-bis(β-hydroxyéthyl)amino]nitrobenzène;
- le 2-N-(β-hydroxyéthyl)amino 5-[N-(β-hydroxyéthyl)amino]nitrobenzène;
- le 2-N-(β-hydroxyéthylamino 5-[N-éthyl,N-(β-hydroxyéthyl)amino] nitrobenzène.

Parmi les colorants nitrobenzéniques dont les nuances ne sont pas comprises entre 2,5 B et 10 RP, on peut citer notamment :

- le 2-amino 5-N-méthylaminonitrobenzène,
- le 2,4-diaminonitrobenzène,
- le 3,4-diaminonitrobenzène,

- le 2,5-diaminonitrobenzène,
- le 3-amino 4-hydroxynitrobenzène,
- le 3-hydroxy 4-aminonitrobenzène,
- le 2-hydroxy 5-aminonitrobenzène,
- le 2-amino 5-hydroxynitrobenzène,
- le 2-amino 3-hydroxynitrobenzène,
- le 2-amino 5-N-(β-hydroxyéthyl)aminonitrobenzène,
- le 2-N-(β-hydroxyéthyl)amino 5-hydroxynitrobenzène,
- le 3-méthoxy 4-N-(β-hydroxyéthyl)aminonitrobenzène,
- le 2-N-méthylamino 4-(β-hydroxyéthyloxy)nitrobenzène,
- le 2-amino 3-méthylnitrobenzène,
- le 2-N-(β-hydroxyéthyl)amino 5-aminonitrobenzène,
- le 2-amino 4-chloro 5-N-(β-hydroxyéthyl)aminonitrobenzène,
- le 2-amino 4-méthyl 5-N-(β-hydroxyéthyl)aminonitrobenzène,
- le 2-amino 4-méthyl 5-N-méthylaminonitrobenzène,
- le 2-N-(β-hydroxyéthyl)amino 5-méthoxynitrobenzène,
- le 2-amino 5-(β-hydroxyéthyloxy)nitrobenzène,
- le 2-N-(β-hydroxyéthyl))aminonitrobenzène,
- le 3-amino 4-N-(β-hydroxyéthyl)aminonitrobenzène,
- le 3-(β-hydroxyéthyloxy)4-N-(β-hydroxyéthyl)aminonitrobenzène,
- le 2-N-méthylamino,4-(β,γ-dihydroxypropyloxy)nitrobenzène,
- le 2-N-(β-hydroxyéthyl)amino 5-(β-hydroxyéthyloxy)nitrobenzène,
- le 2-N-(β-hydroxyéthyl)amino 5-(β,γ-dihydroxypropyloxy)nitrobenzène,
- le 3-hydroxy 4-N-(β-hyroxyéthyl)aminonitrobenzène,
- le 3-N-(β-hydroxyéthyl)amino 4-N-(β-hydroxyéthyl)aminonitrobenzène,
- le 2-amino 4-méthyl 5-N-(β,γ-dihydroxypropyl)aminonitrobenzène,
- le 2-amino 4-méthyl 5-hydroxynitrobenzène,
- le 2-N-(β-aminoéthyl)amino 4-méthoxynitrobenzène,
- le 2-N-(β-aminoéthyl)aminonitrobenzène,
- le 2-N-(β-aminoéthyl)amino 5-N-(β-hydroxyéthyl)aminonitrobenzène,
- le 2-amino 4-méthyl 5-N-(β-aminoéthyl)aminonitrobenzène,
- le 2-amino 4-chloro 5-N-(β-aminoéthyl)aminonitrobenzène,
- le 2-N-(β-hydroxpropyl)amino 5-hydroxynitrobenzène,
- le 2,6-diaminonitrobenzène,
- le 2-N-(β-hydroxyéthyl)amino 6-aminonitrobenzène,
- le 2,6-di(β-hydroxyéthylamino)nitrobenzène,
- le 2-N-éthylamino 5-carboxynitrobenzène,
- l'acide 2-nitro 4-aminodiphénylamine 2'-carboxylique,
- le 3-N-éthylamino 4-hydroxy 5-chloronitrobenzène,
- le 2-N-(β-hydroxyéthyl)amino 5-méthoxy 2,3-dinitrobenzène,
- le 2-N-(β,γ-dihydroxypropyl)amino 5-méthoxy 2,3-dinitrobenzène,
- le 2-N-(β-hydroxyéthyl)amino 5-trifluorométhylnitrobenzène,
- le 2-N-(β,γ-dihydroxypropyl)amino 5-trifluorométhylnitrobenzène,
- le 2-N-(β-hydroxyéthyl)amino 5-méthylnitrobenzène.

Selon un second mode de réalisation, le ou les colorants anthraquinoniques de formule (I) est/sont utilisé(s) selon l'invention en association avec au moins un colorant du type azoïque.

Parmi les colorants azoïques, on peut citer :

- le 1-(4'-aminophénylazo)4-nitrobenzène,
- le 1-(4'-nitrophénylazo)2-méthyl 4-di(β -hydroxyéthyl)aminobenzène,
- le 1-(4'-aminodiphénylazo)2-méthyl 4-di(β -hydroxyéthyl) aminobenzène,
- le 1-[4'-di(β -hydroxyéthyl)aminophénylazo]4-aminobenzène, ou
- le 4-amino 2'-méthyl 4'-N,N-di(β-hydroxyéthyl)aminophénylazobenzène.

Dans les compositions selon l'invention, le ou les colorants anthraquinoniques de formule (I) sont présents dans des proportions allant de 0,01 à 5% en poids du poids total de la composition.

Dans le cas où les anthraquinones selon l'invention sont associées à des colorants nitrés de type benzénique, ceux-ci sont présents dans des proportions allant de 0,01 à 10% du poids total de la composition tinctoriale.

Dans le cas où les anthraquinones selon l'invention sont associées à des colorants azoïques, ceux-ci sont

présents dans des proportions allant de 0,005 à 5% du poids total de la composition tinctoriale.

Selon un troisième mode de réalisation et qui est préféré selon l'invention, le ou les colorants anthraquinoniques de formule (I) selon l'invention, sont utilisés en association avec au moins un colorant nitré du type benzénique décrit ci-dessus et avec au moins un colorant azoïque décrit ci-dessus. Les colorants nitrés benzéniques, azoïques, ou anthraquinoniques selon l'invention, sont alors présents dans les compositions dans les concentrations décrites ci-dessus.

Dans un autre mode de réalisation de l'invention, les composés aminoanthraquinoniques de formule (I) selon l'invention, sont utilisés, en association, avec des précurseurs de colorants d'oxydation et éventuellement avec d'autres colorants directs. Ces précurseurs de colorants d'oxydation sont de préférence choisis parmi les paraphénylènediamines, les paraaminophénols, les bases hétérocycliques telles que par exemple la 2,5 - diaminopyridine ou la 7-aminobenzomorpholine ou les orthoaminophénols.

De plus, ces compositions peuvent contenir, en association avec les précurseurs de colorants d'oxydation ci-dessus, des coupleurs connus dans l'état de la technique. A titre de coupleurs, on peut citer notamment les métadiphénols, les métaaminophénols, les métaphénylènediamines, les métaacylaminophénols, les métauréidophénols, les métacarbalcoxyaminophénols, l'$\alpha$-naphtol, ainsi que les coupleurs possédant un groupement méthylène actif tels que les composés dicétoniques, ainsi que les coupleurs hétérocycliques.

Dans cette forme de réalisation de l'invention, les précurseurs de colorants d'oxydation sont utilisés à des concentrations comprises entre 0,01 et 10% en poids, et de préférence entre 0,03 et 5% en poids, sur la base du poids total de la composition. Les coupleurs, quant à eux, peuvent être présents dans ces compositions, dans des proportions allant de 0,001 à 8% en poids, et de préférence entre 0,015 et 5% en poids.

Dans cette forme de réalisation où des précurseurs de colorants d'oxydation sont présents, les compositions peuvent en outre contenir des agents réducteurs ou antioxydants, qui sont alors présents dans des proportions comprises entre 0,05 et 3% en poids par rapport au poids total de la composition.

Lorsque les compositions tinctoriales selon l'invention constituent des teintures d'oxydation, c'est-à-dire comprennent des précurseurs de colorants d'oxydation, et donc impliquent la révélation par un oxydant, le colorant anthraquinonique selon l'invention est essentiellement utilisé en vue d'apporter des reflets à la teinture finale.

Les compositions selon l'invention peuvent en outre contenir également d'autres colorants aminoanthraquinoniques et notamment le 1,4-(di-$\beta$ -hydroxyéthylamino)9,10-anthraquinone et les colorants nitropyridiniques.

Les compositions selon l'invention peuvent comprendre comme support approprié à leur application, l'eau et/ou des solvants organiques cosmétiquement acceptables, et plus particulièrement des alcools tels que l'alcool éthylique, l'alcool isopropylique, l'alcool benzylique et l'alcool phényléthylique, ou des glycols ou éthers de glycol tels que, par exemple, l'éthylèneglycol et ses éthers monométhylique, monoéthylique et monobutylique, le propylène glycol, le butylèneglycol, le dipropylèneglycol, ainsi que les alkyléthers du diéthylèneglycol, comme par exemple le monométhyl éther ou le monobutyléther du diéthylèneglycol. La concentration de ces solvants organiques est alors par exemple comprise entre 0,5 et 20%, et de préférence 2 et 10% en poids par rapport au poids total de la composition.

On peut également ajouter à la composition selon l'invention des amides gras tels que les mono- et diéthanolamides des acides dérivés du coprah, de l'acide laurique ou de l'acide oléique, à des concentrations comprises entre 0,05 et 10% en poids.

On peut également ajouter à la composition selon l'invention, des agents tensio-actifs anioniques, cationiques, non-ioniques, amphotères ou leurs mélanges. De préférence, les tensio-actifs sont présents dans la composition selon l'invention à une proportion comprise entre 0,1 et 50% en poids, et de préférence entre 1 et 20% en poids par rapport au poids total de la composition.

Comme produits épaississants que l'on peut éventuellement ajouter à la composition selon l'invention, on peut citer notamment l'alginate de sodium, la gomme arabique, la gomme de guar, les gommes de xanthane et les dérivés de la cellulose, ainsi que les polymères d'acide acrylique.

On peut également utiliser des agents épaississants minéraux, tels que la bentonite. Ces épaississants sont utilisés seuls ou en mélange, et de préférence sont présents en une proportion comprise entre 0,5 et 5% en poids par rapport au poids total de la composition, et avantageusement, entre 0,5 et 3% en poids.

Les compositions selon l'invention peuvent être formulées à pH acide, neutre ou alcalin, le pH pouvant varier entre 4 et 10,5, et de préférence entre 6 et 10.

Parmi les agents d'alcalinisation qui peuvent être utilisés, on peut mentionner les alcanolamines, les hydroxydes et les carbonates alcalins ou l'hydroxyde d'ammonium.

Parmi les agents d'acidification qui peuvent être utilisés, on peut mentionner l'acide lactique, l'acide acétique, l'acide tartrique, l'acide phosphorique, l'acide chlorhydrique et l'acide citrique.

Les compositions tinctoriales peuvent contenir en outre divers adjuvants usuels tels que des parfums, des

agents séquestrants, des produits filmogènes et des agents de traitement des cheveux, des agents dispersants, des agents de conditionnement du cheveu, des agents conservateurs, des opacifiants, ainsi que tout autre adjuvant utilisé habituellement en cosmétique.

Les compositions selon l'invention peuvent se présenter sous les diverses formes usuelles pour le traitement des fibres kératiniques, telles que des liquides, épaissis ou gélifiés, des crèmes, des mousses ou sous toutes autres formes appropriées pour réaliser une telle teinture.

La présente invention concerne également un procédé de teinture des fibres kératiniques humaines, dans lequel on applique sur les fibres kératiniques, une composition selon l'invention, pour la coloration directe.

Elle concerne également un procédé de coloration d'oxydation, dans lequel on applique une composition selon l'invention comportant au moins un précurseur de colorant d'oxydation, et simultanément ou ensuite, un agent oxydant.

Ainsi, si la composition tinctoriale contient des colorants d'oxydation, on peut la mélanger au moment de l'emploi avec un agent oxydant. Cet agent oxydant est usuellement de l'eau oxygénée titrant de 10 à 40 volumes. L'application peut être ainsi simultanée ou encore séquentielle, c'est-à-dire que la composition renfermant l'agent oxydant est appliquée après la composition tinctoriale contenant les colorants d'oxydation.

Dans le cas où la composition selon l'invention contient des colorants d'oxydation, on applique la composition ou les compositions sur les cheveux pendant 1 à 60 minutes, et de préférence 10 à 45 minutes, puis on rince.

Dans le cas où la composition tinctoriale ne comporte que des colorants directs, elle est appliquée sur des cheveux pendant un temps de pose variant entre 1 et 60 minutes, et de préférence 5 à 45 minutes, puis on rince.

Dans chacun des deux cas ci-dessus, on peut éventuellement laver, rincer à nouveau et sécher les cheveux.

Les compositions tinctoriales conformes à l'invention peuvent être appliquées sur des cheveux naturels ou teints, permanentés ou non, ou sur des cheveux fortement ou légèrement décolorés et, éventuellement, permanentés.

La présente invention concerne également des kits de coloration ou dispositifs à plusieurs composants comportant au moins un compartiment contenant la composition selon l'invention où des colorants d'oxydation sont présents, et l'autre comportant l'agent oxydant.

D'autres avantages et caractéristiques de l'invention apparaîtront à la lecture des exemples ci-après, qui sont indiqués à titre illustratif.

EXEMPLE 1

| | | |
|---|---|---|
| - 1,4-di($\beta$,$\gamma$-dihydroxypropylamino) 9,10-anthraquinone | | 0,25 g |
| - 2-butoxyéthanol | | 10,0 g |
| - Hydroxyéthylcellulose ("Cellosize WP03H" de la Société UNION CARBIDE) | | 2,0 g |
| - Lauryl éther sulfate de sodium à 2 moles d'oxyde d'éthylène ("Sactipon 8533" de la Société LEVER) | | 3,0 g MA |
| - 2-amino 2-méthyl 1-propanol qs pH=9,5 | | |
| - Eau déminéralisée | qsp | 100,0 g |

Cette composition appliquée pendant un temps de pose de 30 minutes sur des cheveux gris naturels à 90% de blancs, leur confère après rinçage et séchage, une nuance bleue.

EXEMPLE 2

| | | |
|---|---|---|
| - 1,4-di(β,γ-dihydroxypropylamino) 9,10-anthraquinone | | 0,2 g |
| - 4-amino 2'-méthyl 4'-N,N-di-(β-hydroxyéthyl)aminophénylazobenzène | | 0,1 g |
| - 2-éthoxyéthanol | | 10,0 g |
| - Nonylphénol à 9 moles d'oxyde d'éthylène ("Simulsol 830 NP" de la Société SEPPIC) | | 2,0 g |
| - Triéthanolamine qs pH=6,5 | | |
| - Eau déminéralisée | qsp | 100,0 g |

On applique cette composition sur des cheveux gris naturels à 90% de blancs, qu'on laisse poser durant 20 minutes. On obtient après rinçage et séchage une coloration verte.

EXEMPLE 3

| | | |
|---|---|---|
| - 1,4-di(β,γ-dihydroxypropylamino) 9,10-anthraquinone | | 0,5 g |
| - 4-amino 2'-méthyl 4'-N,N-di(β-hydroxyéthyl) aminophénylazobenzène | | 0,1 g |
| - 2-N(β-hydroxyéthyl)amino 5-N,N-di (β-hydroxyéthyl)aminonitrobenzène | | 1,0 g |
| - 2-N-méthylamino 5-N-méthyl N-β,γ-di(hydroxypropyl)aminonitrobenzène | | 0,5 g |
| - 2-N-méthylamino 4-β,γ-dihydroxy-propyloxynitrobenzène | | 0,5 g |
| - 2-N-méthylamino 4-(β-hydroxyéthyl)amino nitrobenzène | | 0,1 g |
| - Diéthanolamide oléïque | | 3,0 g |
| - Acide laurique | | 1,0 g |
| - 2-éthoxyéthanol | | 5,0 g |
| - "Cellosize WPO 3H" (UNION CARBIDE) | | 2,0 g |
| - 2-amino 2-méthyl 1-propanol qs pH=9,5 | | |
| - Eau déminéralisée | qsp | 100,0 g |

Cette composition est appliquée et laissée posée 20 minutes sur des cheveux permanentés gris à 90% de blancs. Après rinçage et séchage, ces cheveux sont colorés dans une nuance châtain foncé.

EXEMPLE 4

| | | | |
|---|---|---|---|
| - 1,4-di(β,γ-dihydroxypropylamino) 9,10-anthraquinone | | 0,3 | g |
| - 2-méthyl 1,4-diaminobenzène | | 0,8 | g |
| - p-aminophénol | | 0,05 | g |
| - 1-méthyl 2-hydroxy 4-β-hydroxyéthyl-aminobenzène | | 0,95 | g |
| - m-aminophénol | | 0,3 | g |
| - Gomme de xanthane, vendue par la Société KELCO sous la dénomination "KELTROL T" | | 2,2 | g |
| - Alkyl éther sulfate de sodium à 28% de MA, vendu sous la dénomination "SACTIPON 8533" par la Société LEVER | | 20,0 | g |
| - Diéthanolamide oléique | | 2,5 | g |
| - Acide éthylènediaminotétracétique | | 0,1 | g |
| - Acide thiolactique | | 0,3 | g |
| - Ammoniaque à 20% de $NH_3$ | | 12,0 | g |
| - Eau | qsp | 100,0 | g |

Cette composition gélifiée est diluée au moment de l'emploi avec un poids égal d'$H_2O_2$ à 20 volumes. Le mélange obtenu est appliqué et laissé posé pendant 30 minutes sur des cheveux bruns naturels.

On obtient après rinçage, shampooing et séchage, une nuance châtain violine.

## Revendications

**Revendications pour les Etats contractants suivants : AT, BE, CH, DE, DK, GB, GR, IT, LI, NL, SE**

**1.** Utilisation des anthraquinones de formule (I) :

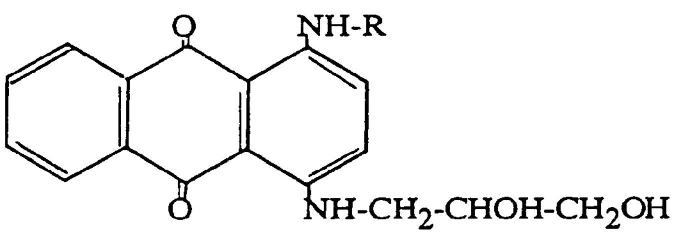

(I)

où R représente un radical monohydroxyalkyle en $C_2$ à $C_6$, ou un radical polyhydroxyalkyle en $C_3$ à $C_6$, pour la teinture des fibres kératiniques humaines.

**2.** Utilisation selon la revendication 1, caractérisée en ce que l'anthraquinone est la 1,4-di-(β,γ-dihydroxypropylamino)9,10-anthraquinone ou la 1-(β-hydroxyéthylamino)4-(β,γ-dihydroxypropylamino) 9,10-anthraquinone.

3. Composition cosmétique, caractérisée en ce qu'elle comporte une anthraquinone de formule (I) :

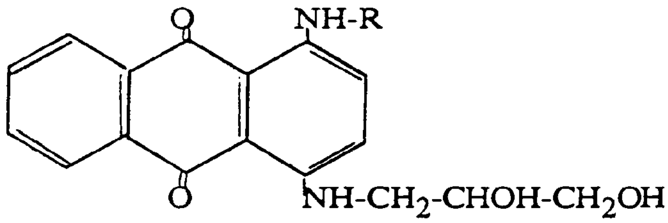

(I)

où R représente un radical monohydroxyalkyle en $C_2$-$C_6$ ou un radical polyhydroxyalkyle en $C_3$-$C_6$ dans un support cosmétiquement acceptable, et en outre l'un au moins des adjuvants cosmétiques choisis parmi les agents tensio-actifs anioniques, cationiques, nonioniques, amphotères ou leurs mélanges, les épaississants, les agents dispersants, les agents de pénétration, les séquestrants, les agents filmogènes, les tampons, les parfums, les agents alcalinisants ou acidifiants, les agents opacifiants, les agents de traitement des cheveux ou les conservateurs.

4. Composition cosmétique selon la revendication 3, caractérisée en ce que R représente $-CH_2-CHOH-CH_2OH$ ou $CH_2-CH_2OH$.

5. Composition tinctoriale pour fibres kératiniques, caractérisée en ce qu'elle comporte au moins une anthraquinone de formule (I) :

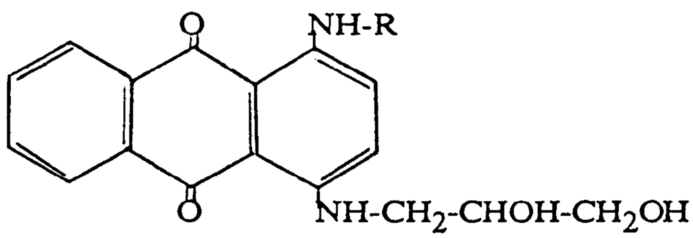

(I)

où R représente un radical monohydroxyalkyle en $C_2$-$C_6$ ou polyhydroxyalkyle en $C_3$-$C_6$, en association avec un ou plusieurs autres colorants choisis parmi :
(i) les colorants nitrés du type benzénique;
(ii) les colorants du type azoïque;
(iii) les précurseurs de colorants d'oxydation,
dans un support cosmétiquement acceptable.

6. Composition selon la revendication 5, caractérisée en ce que l'anthraquinone de formule (I) est celle où R représente $-CH_2-CHOH-CH_2OH$ ou $CH_2-CH_2OH$.

7. Composition selon la revendication 5 ou 6, caractérisée en ce que les colorants nitrés du type benzénique sont choisis parmi les colorants bleus ou violets du type nitroparaphénylènediamine, ayant une nuance selon Munsell comprise entre 2,5 B et 10 RP.

8. Composition selon la revendication 7, caractérisée en ce que les colorants benzéniques du type nitré sont choisis parmi :
- le 2-N-méthylamino 5-[N,N-bis(β-hydroxyéthyl)amino]nitrobenzène;
- le 2-N-méthylamino 5-[N-méthyl,N-(β-hydroxyéthyl)amino]nitrobenzène;
- le 2-(N-β-hydroxyéthyl)amino 5-[N,N-bis(β-hydroxyéthyl)amino] nitrobenzène;
- le 2-(N-β-hydroxyéthyl)amino 5-[N-méthyl,N-(β-hydroxyéthyl)amino] nitrobenzène;
- le 2-(N-γ-hydroxypropylamino) 5-[N,N-bis(β-hydroxyéthyl)amino] nitrobenzène;
- le 2-(N-β-aminoéthylamino) 5-[N,N-bis(β-hydroxyéthyl)amino]nitrobenzène;
- le 2-N-méthylamino 5-[N-méthyl,N-(β,γ-dihydroxypropyl)amino] nitrobenzène;
- le 2-amino 5-[N,N-bis(β-hydroxyéthyl)amino]nitrobenzène;

- le 2-N-(β-hydroxyéthyl)amino 5-[N-(β-hydroxyéthyl)amino]nitrobenzène;
le 2-N-(β-hydroxyéthylamino 5-[N-éthyl,N-(β-hydroxyéthyl)amino] nitrobenzène.

**9.** Composition selon l'une des revendications 5 à 8, caractérisée en ce que les colorants nitrés du type benzénique sont choisis parmi les nitrophénylènediamines jaunes, oranges ou rouges, les nitroaminophénols, les nitroaminoalcoxybenzènes, ou les nitroaminohydroxyalcoxybenzènes.

**10.** Composition selon la revendication 9, caractérisée en ce que les colorants nitrés du type benzénique sont choisis parmi :

- le 2-amino 5-N-méthylaminonitrobenzène,
- le 2,4-diaminonitrobenzène,
- le 3,4-diaminonitrobenzène,
- le 2,5-diaminonitrobenzène,
- le 3-amino 4-hydroxynitrobenzène,
- le 3-hydroxy 4-aminonitrobenzène,
- le 2-hydroxy 5-aminonitrobenzène,
- le 2-amino 5-hydroxynitrobenzène,
- le 2-amino 3-hydroxynitrobenzène,
- le 2-amino 5-N-(β-hydroxyéthyl)aminonitrobenzène,
- le 2-N-(β-hydroxyéthyl)amino 5-hydroxynitrobenzène,
- le 3-méthoxy 4-N-(β-hydroxyéthyl)aminonitrobenzène,
- le 2-N-méthylamino 4-(β-hydroxyéthyloxy)nitrobenzène,
- le 2-amino 3-méthylnitrobenzène,
- le 2-N-(β-hydroxyéthyl)amino 5-aminonitrobenzène,
- le 2-amino 4-chloro 5-N-(β-hydroxyéthyl)aminonitrobenzène,
- le 2-amino 4-méthyl 5-N-(β-hydroxyéthyl)aminonitrobenzène,
- le 2-amino 4-méthyl 5-N-méthylaminonitrobenzène,
- le 2-N-(β-hydroxyéthyl)amino 5-méthoxynitrobenzène,
- le 2-amino 5-(β-hydroxyéthyloxy)nitrobenzène,
- le 2-N-(β-hydroxyéthyl))aminonitrobenzène,
- le 3-amino 4-N-(β-hydroxyéthyl)aminonitrobenzène,
- le 3-(β-hydroxyéthyloxy)4-N-(β-hydroxyéthyl)aminonitrobenzène,
- le 2-N-méthylamino,4-(β,γ-dihydroxypropyloxy)nitrobenzène,
- le 2-N-(β-hydroxyéthyl)amino 5-(β-hydroxyéthyloxy)nitrobenzène,
- le 2-N-(β-hydroxyéthyl)amino 5-(β,γ-dihydroxypropyloxy)nitrobenzène,
- le 3-hydroxy 4-N-(β-hyroxyéthyl)aminonitrobenzène,
- le 3-N-(β-hydroxyéthyl)amino 4-N-(β-hydroxyéthyl)aminonitrobenzène,
- le 2-amino 4-méthyl 5-N-(β,γ-dihydroxypropyl)aminonitrobenzène,
- le 2-amino 4-méthyl 5-hydroxynitrobenzène,
- le 2-N-(β-aminoéthyl)amino 4-méthoxynitrobenzène,
- le 2-N-(β-aminoéthyl)aminonitrobenzène,
- le 2-N-(β-aminoéthyl)amino 5-N-(β-hydroxyéthyl)aminonitrobenzène,
- le 2-amino 4-méthyl 5-N-(β-aminoéthyl)aminonitrobenzène,
- le 2-amino 4-chloro 5-N-(β-aminoéthyl)aminonitrobenzène,
- le 2-N-(β-hydroxpropyl)amino 5-hydroxynitrobenzène,
- le 2,6-diaminonitrobenzène,
- le 2-N-(β-hydroxyéthyl)amino 6-aminonitrobenzène,
- le 2,6-di(β-hydroxyéthylamino)nitrobenzène,
- le 2-N-éthylamino 5-carboxynitrobenzène,
- l'acide 2-nitro 4-aminodiphénylamine 2'-carboxylique,
- le 3-N-éthylamino 4-hydroxy 5-chloronitrobenzène,
- le 2-N-(β-hydroxyéthyl)amino 5-méthoxy 2,3-dinitrobenzène,
- le 2-N-(β,γ-dihydroxypropyl)amino 5-méthoxy 2,3-dinitrobenzène,
- le 2-N-(β-hydroxyéthyl)amino 5-trifluorométhylnitrobenzène,
- le 2-N-(β,γ-dihydroxypropyl)amino 5-trifluorométhylnitrobenzène,
- le 2-N-(β-hydroxyéthyl)amino 5-méthylnitrobenzène.

**11.** Composition selon l'une des revendications 5 à 10, caractérisée en ce qu'elle comporte en outre des colorants nitrés du type nitropyridinique ou d'autres composés anthraquinoniques.

**12.** Composition selon l'une des revendications 5 à 11, caractérisée en ce que le colorant azoïque est choisi parmi :
- le 1-(4'-aminophénylazo)4-nitrobenzène,
- le 1-(4'-nitrophénylazo)2-méthyl 4-di(β -hydroxyéthyl)aminobenzène,
- le 1-(4'-aminodiphénylazo)2-méthyl 4-di(β-hydroxyéthyl) aminobenzène,
- le 1-[4'-di(β-hydroxyéthyl)aminophénylazo]4-aminobenzène, ou
- le 4-amino 2'-méthyl 4'-N,N-di(β-hydroxyéthyl)aminophénylazobenzène.

**13.** Composition selon l'une des revendications 5 à 12, caractérisée en ce que les précurseurs de colorants d'oxydation sont choisis parmi les paraphénylènediamines, les paraaminophénols, les bases hétérocycliques, les orthoaminophénols.

**14.** Composition selon l'une des revendications 5 à 13, caractérisée en ce qu'elle comporte les coupleurs choisis parmi les métadiphénols, les métaaminophénols, les métaphénylènediamines, les métaacylaminophénols, les métaauréidophénols, les métacarbalcoxyaminophénols, l'alpha-naphtol, les composés dicétoniques et les coupleurs hétérocycliques.

**15.** Composition selon l'une des revendications 5 à 14, caractérisée en ce qu'elle comporte de 0,01 à 5% en poids de colorant(s) anthraquinonique(s).

**16.** Composition selon l'une des revendications 5 à 15, caractérisée en ce qu'elle comporte entre 0,01 et 10% en poids de colorant(s) nitré(s) du type benzénique.

**17.** Composition selon l'une des revendications 5 à 16, caractérisée en ce qu'elle comporte entre 0,005 et 5% en poids de colorant(s) azoïque(s).

**18.** Composition selon l'une des revendications 5 à 17, caractérisée en ce qu'elle comporte entre 0,01 et 10% en poids de précurseurs de colorants d'oxydation, de préférence entre 0,03 et 5% en poids.

**19.** Composition selon l'une des revendications 5 à 18, caractérisée en ce qu'elle comporte entre 0,001 et 8% en poids de coupleurs, de préférence entre 0,015 et 5 % en poids.

**20.** Composition selon l'une des revendications 5 et 13 à 19, caractérisée en ce qu'elle comporte en outre des agents réducteurs ou antioxydants dans des proportions comprises entre 0,05 et 3% en poids du poids total de la composition.

**21.** Composition selon l'une des revendications 5 à 20, caractérisée en ce qu'elle comporte en outre des adjuvants cosmétiques choisis parmi les agents tensio-actifs anioniques, cationiques, nonioniques, amphotères ou leurs mélanges, les épaississants, les agents dispersants, les agents de pénétration, les séquestrants, les agents filmogènes, les tampons, les parfums, les agents alcalinisants ou acidifiants, les agents nacrants ou les agents solubilisants.

**22.** Procédé de coloration directe des fibres kératiniques, caractérisé en ce que l'on applique sur les fibres kératiniques une composition telle que définie dans l'une des revendications 3 à 12, 15 à 17 et 21.

**23.** Procédé de coloration d'oxydation des fibres kératiniques, caractérisé en ce que l'on applique sur les fibres kératiniques une composition selon l'une des revendications 3 à 21, l'application de cette composition étant accompagnée ou suivie de l'application d'une composition comportant un agent oxydant dans un milieu cosmétiquement acceptable.

**24.** Kit de teinture des fibres kératiniques, caractérisé en ce qu'il comporte au moins un compartiment contenant une composition selon l'une des revendications 3 à 21 et au moins un compartiment contenant un agent d'oxydation dans un solvant cosmétiquement acceptable.

**Revendications pour l'Etat contractant suivant : ES**

**1.** Utilisation des anthraquinones de formule (I) :

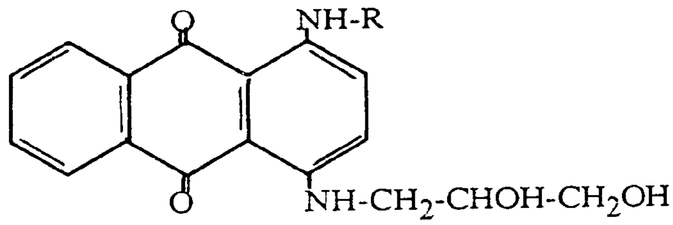

(I)

où R représente un radical monohydroxyalkyle en $C_2$ à $C_6$, ou un radical polyhydroxyalkyle en $C_3$ à $C_6$, pour la teinture des fibres kératiniques humaines.

2. Utilisation selon la revendication 1, caractérisée en ce que l'anthraquinone est la 1,4-di-(β,γ-dihydroxypropylamino)9,10-anthraquinone ou la 1-(β-hydroxyéthylamino)4-(β,γ-dihydroxypropylamino) 9,10-anthraquinone.

3. Composition cosmétique, caractérisée en ce qu'elle comporte une anthraquinone de formule (I) :

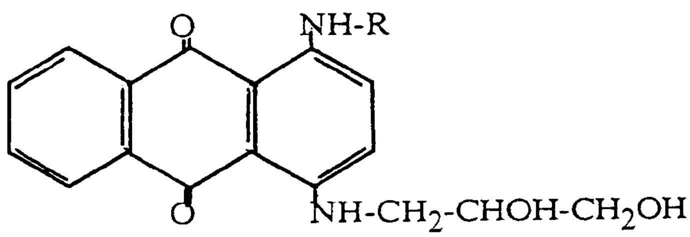

(I)

où R représente un radical monohydrouyalkyle en $C_2$-$C_6$ ou un radical polyhydroxyalkyle en $C_3$-$C_6$ dans un support cosmétiquement acceptable, et en outre l'un au moins des adjuvants cosmétiques choisis parmi les agents tensio-actifs anioniques, cationiques, non-ioniques, amphotères ou leurs mélanges, les épaississants, les agents dispersants, les agents de pénétration, les séquestrants, les agents filmogènes, les tampons, les parfums, les agents alcalinisants ou acidifiants, les agents opacifiants, les agents de traitement des cheveux ou les conservateurs.

4. Composition cosmétique selon la revendication 3, caractérisée en ce que R représente $-CH_2-CHOH-CH_2OH$ ou $CH_2-CH_2OH$.

5. Composition tinctoriale pour fibres kératiniques, caractérisée en ce qu'elle comporte au moins une anthraquinone de formule (I) :

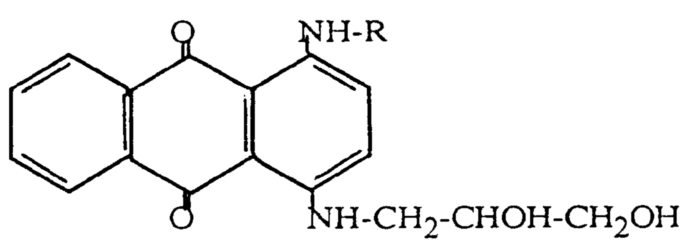

(I)

où R représente un radical monohydroxyalkyle en $C_2$-$C_6$ ou polyhydroxyalkyle en $C_3$-$C_6$, en association avec un ou plusieurs autres colorants choisis parmi :
(i) les colorants nitrés du type benzénique;
(ii) les colorants du type azoïque;
(iii) les précurseurs de colorants d'oxydation,
dans un support cosmétiquement acceptable.

6. Composition selon la revendication 5, caractérisée en ce que l'anthraquinone de formule (I) est celle où

R représente $-CH_2-CHOH-CH_2OH$ ou $CH_2-CH_2OH$.

7. Composition selon la revendication 5 ou 6, caractérisée en ce que les colorants nitrés du type benzénique sont choisis parmi les colorants bleus ou violets du type nitroparaphénylènediamine, ayant une nuance selon Munsell comprise entre 2,5 B et 10 RP.

8. Composition selon la revendication 7, caractérisée en ce que les colorants benzéniques du type nitré sont choisis parmi :
   - le 2-N-méthylamino 5-[N,N-bis(β-hydroxyéthyl)amino]nitrobenzène;
   - le 2-N-méthylamino 5-[N-méthyl,N-(β-hydroxyéthyl)amino]nitrobenzène;
   - le 2-(N-β-hydroxyéthyl)amino 5-[N,N-bis(β-hydroxyéthyl)amino] nitrobenzène;
   - le 2-(N-β-hydroxyéthyl)amino 5-[N-méthyl,N-(β-hydroxyéthyl)amino] nitrobenzène;
   - le 2-(N-γ-hydroxypropylamino) 5-[N,N-bis(β-hydroxyéthyl)amino] nitrobenzène;
   - le 2-(N-β-aminoéthylamino) 5-[N,N-bis(β-hydroxyéthyl)amino]nitrobenzène;
   - le 2-N-méthylamino 5-[N-méthyl,N-(β,γ-dihydroxypropyl)amino] nitrobenzène;
   - le 2-amino 5-[N,N-bis(β-hydroxyéthyl)amino]nitrobenzène;
   - le 2-N-(β-hydroxyéthyl)amino 5-[N-(β-hydroxyéthyl)amino]nitrobenzène;
   - le 2-N-(β-hydroxyéthylamino 5-[N-éthyl,N-(β-hydroxyéthyl)amino] nitrobenzène.

9. Composition selon l'une des revendications 5 à 8, caractérisée en ce que les colorants nitrés du type benzénique sont choisis parmi les nitrophénylènediamines jaunes, oranges ou rouges, les nitroaminophénols, les nitroaminoalcoxybenzènes, ou les nitroaminohydroxyalcoxybenzènes.

10. Composition selon la revendication 9, caractérisée en ce que les colorants nitrés du type benzénique sont choisis parmi :
   - le 2-amino 5-N-méthylaminonitrobenzène,
   - le 2,4-diaminonitrobenzène,
   - le 3,4-diaminonitrobenzène,
   - le 2,5-diaminonitrobenzène,
   - le 3-amino 4-hydroxynitrobenzène,
   - le 3-hydroxy 4-aminonitrobenzène,
   - le 2-hydroxy 5-aminonitrobenzène,
   - le 2-amino 5-hydroxynitrobenzène,
   - le 2-amino 3-hydroxynitrobenzène,
   - le 2-amino 5-N-(β-hydroxyéthyl)aminonitrobenzène,
   - le 2-N-(β-hydroxyéthyl)amino 5-hydroxynitrobenzène,
   - le 3-méthoxy 4-N-(β-hydroxyéthyl)aminonitrobenzène,
   - le 2-N-méthylamino 4-(β-hydroxyéthyloxy)nitrobenzène,
   - le 2-amino 3-méthylnitrobenzène,
   - le 2-N-(β-hydroxyéthyl)amino 5-aminonitrobenzène,
   - le 2-amino 4-chloro 5-N-(β-hydroxyéthyl)aminonitrobenzène,
   - le 2-amino 4-méthyl 5-N-(β-hydroxyéthyl)aminonitrobenzène,
   - le 2-amino 4-méthyl 5-N-méthylaminonitrobenzène,
   - le 2-N-(β-hydroxyéthyl)amino 5-méthoxynitrobenzène,
   - le 2-amino 5-β-hydroxyéthyloxy)nitrobenzène,
   - le 2-N-(β-hydroxyéthyl)aminonitrobenzène,
   - le 3-amino 4-N-(β-hydroxyéthyl)aminonitrobenzène,
   - le 3-(β-hydroxyéthyloxy)4-N-(β-hydroxyéthyl)aminonitrobenzène,
   - le 2-N-méthylamino,4-(β,γ-dihydroxypropyloxy)nitrobenzène,
   - le 2-N-(β-hydroxyéthyl)amino 5-(β--hydroxyéthyloxy)nitrobenzène,
   - le 2-N-(β-hydroxyéthyl)amino 5-(β,γ-dihydroxypropyloxy)nitrobenzène,
   - le 3-hydroxy 4-N-(β-hyroxyéthyl)aminonitrobenzène,
   - le 3-N-(β-hydroxyéthyl)amino 4-N-(β-hydroxyéthyl)aminonitrobenzène,
   - le 2-amino 4-méthyl 5-N-(β,γ-dihydroxypropyl)aminonitrobenzène,
   - le 2-amino 4-méthyl 5-hydroxynitrobenzène,
   - le 2-N-(β-aminoéthyl)amino 4-méthoxynitrobenzène,
   - le 2-N-(β-aminoéthyl)aminonitrobenzène,
   - le 2-N(β-aminoéthyl)amino 5-N-(β-hydroxyéthyl)aminonitrobenzène,

- le 2-amino 4-méthyl 5-N-(β-aminoéthyl)aminonitrobenzène,
- le 2-amino 4-chloro 5-N-(β-aminoéthyl)aminonitrobenzène,
- le 2-N-(β-hydroxypropyl)amino 5-hydroxynitrobenzène,
- le 2,6-diaminonitrobenzène,
- le 2-N-(β-hydroxyéthyl)amino 6-aminonitrobenzène,
- le 2,6-di(β-hydroxyétylamino)nitrobenzène,
- le 2-N-éthylamino 5-carboxynitrobenzène,
- l'acide 2-nitro 4-aminodiphénylamine 2'-carboxylique,
- le 3-N-éthylamino 4-hydroxy 5-chloronitrobenzène,
- le 2-N-(β-hydroxyéthyl)amino 5-méthoxy 2,3-dinitrobenzène,
- le 2-N-(β,γ-dihydroxypropyl)amino 5-méthoxy 2,3-dinitrobenzène,
- le 2-N-(β-hydroxyéthyl)amino 5-trifluorométhylnitrobenzène,
- le 2-N-(β,γ-dihydroxypropyl)amino 5-trifluorométhylnitrobenzène,
- le 2-N-(β-hydroxyéthyl)amino 5-méthylnitrobenzène.

**11.** Composition selon l'une des revendications 5 à 10, caractérisée en ce qu'elle comporte en outre des colorants nitrés du type nitropyridinique ou d'autres composés anthraquinoniques.

**12.** Composition selon l'une des revendications 5 à 11, caractérisée en ce que le colorant azoique est choisi parmi :
- le 1-(4'-aminophénylazo)4-nitrobenzène,
- le 1-(4'-nitrophénylazo)2-méthyl 4-di(β-hydroxyéthyl)aminobenzène,
- le 1-(4'-aminodiphénylazo)2-méthyl 4-di(β-hydroxyéthyl) aminobenzène,
- le 1-[4'-di(β-hydroxyéthyl)aminophénylazo]4-aminobenzène, ou
- le 4-amino 2'-méthyl 4'-N,N-di(β-hydroxyéthyl)aminophénylazobenzène.

**13.** Composition selon l'une des revendications 5 à 12, caractérisée en ce que les précurseurs de colorants d'oxydation sont choisis parmi les paraphénylènediamines, les paraaminophénols, les bases hétérocycliques, les orthoaminophénols.

**14.** Composition selon l'une des revendications 5 à 13, caractérisée en ce qu'elle comporte les coupleurs choisis parmi les métadiphénols, les métaaminophénols, les métaphénylènediamines, les métaacylaminophénols, les métauréidophénols, les métacarbalcoxyaminophénols, l'alpha-naphtol, les composés dicétoniques et les coupleurs hétérocycliques.

**15.** Composition selon l'une des revendications 5 à 14, caractérisée en ce qu'elle comporte de 0,01 à 5% en poids de colorant(s) anthraquinonique(s).

**16.** Composition selon l'une des revendications 5 à 15, caractérisée en ce qu'elle comporte entre 0,01 et 10% en poids de colorant(s) nitré(s) du type benzénique.

**17.** Composition selon l'une des revendications 5 à 16, caractérisée en ce qu'elle comporte entre 0,005 et 5% en poids de colorant(s) azoïque(s).

**18.** Composition selon l'une des revendications 5 à 17, caractérisée en ce qu'elle comporte entre 0,01 et 10% en poids de précurseurs de colorants d'oxydation, de préférence entre 0,03 et 5% en poids.

**19.** Composition selon l'une des revendications 5 à 18, caractérisée en ce qu'elle comporte entre 0,001 et 8% en poids de coupleurs, de préférence entre 0,015 et 5% en poids.

**20.** Composition selon l'une des revendications 5 et 13 à 19, caractérisée en ce qu'elle comporte en outre des agents réducteurs ou antioxydants dans des proportions comprises entre 0,05 et 3% en poids du poids total de la composition.

**21.** Composition selon l'une des revendications 5 à 20, caractérisée en ce qu'elle comporte en outre des adjuvants cosmétiques choisis parmi les agents tensio-actifs anioniques, cationiques, non-ioniques, amphotères ou leurs mélanges, les épaississants, les agents dispersants, les agents de pénétration, les séquestrants, les agents filmogènes, les tampons, les parfums, les agents alcalinisants ou acidifiants, les agents nacrants ou les agents solubilisants.

**22.** Procédé de coloration directe des fibres kératiniques, caractérisé en ce que l'on applique sur les fibres kératiniques une composition telle que définie dans l'une des revendications 3 à 12, 15 à 17 et 21.

**23.** Procédé de coloration d'oxydation des fibres kératiniques, caractérisé en ce que l'on applique sur les fibres kératiniques une composition selon l'une des revendications 3 à 21, l'application de cette composition étant accompagnée ou suivie de l'application d'une composition comportant un agent oxydant dans un milieu cosmétiquement acceptable.

**24.** Kit de teinture des fibres kératiniques, caractérisé en ce qu'il comporte au moins un compartiment contenant une composition selon l'une des revendications 3 à 21 et au moins un compartiment contenant un agent d'oxydation dans un solvant cosmétiquement acceptable.

**25.** Procédé de préparation d'une composition cosmétique, caractérisé en ce que l'on incorpore au moins une anthraquinone de formule (I):

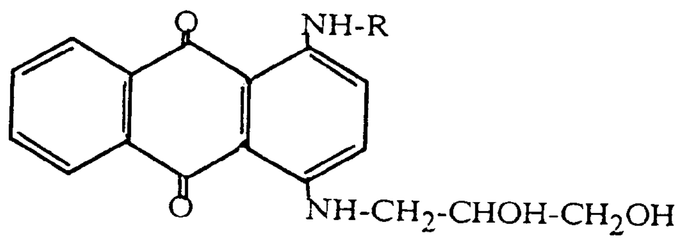

(I)

où R représente un radical monohydroxyalkyle en $C_2$- $C_6$ ou un radical polyhydroxyalkyle en $C_3$-$C_6$, à une concentration comprise entre 0,01 et 5% en poids par rapport au poids total de la composition dans un support cosmétiquement acceptable comportant en outre l'un au moins des adjuvants cosmétiques choisis parmi les agents tensio-actifs anioniques, cationiques, non-ioniques, amphotères ou leurs mélanges, les épaississants, les agents dispersants, les agents de pénétration, les séquestrants, les agents filmogènes, les tampons, les parfums, les agents alcalinisants ou acidifiants, les agents opacifiants, les agents de traitement des cheveux ou les conservateurs.

## Patentansprüche

### Patentansprüche für folgende Vertragsstaaten : AT, BE, CH, DE, DK, GB, GR, IT, LI, NL, SE

**1.** Verwendung von Anthrachinon der Formel (I) :

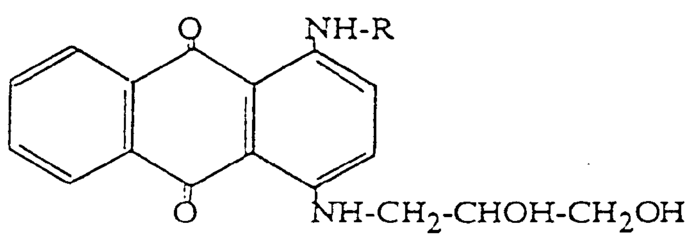

(I)

worin R einen $C_{2-6}$-Monohydroxyalkyl- oder $C_{3-6}$-Polyhydroxyalkylrest bedeutet, zur Färbung keratinischer menschlicher Fasern.

**2.** Verwendung gemäß Anspruch 1,
dadurch **gekennzeichnet,** daß
das Anthrachinon 1,4-Di(β,y-dihydroxypropylamino)-9,10-anthrachinon oder 1-(β-Hydroxyethylamino)-4-(β,γ-dihydroxypropylamino) -9,10-anthrachinon ist.

**3.** Kosmetische Zusammensetzung,
dadurch **gekennzeichnet,** daß

sie ein Anthrachinon der Formel (I) :

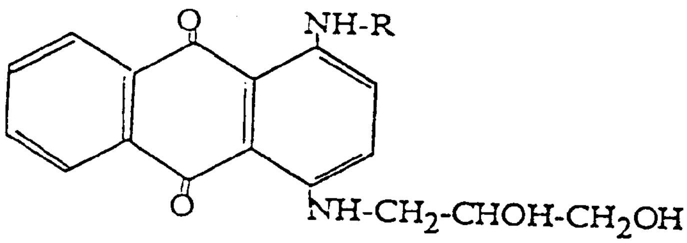

(I)

worin R einen $C_2$-$C_6$-Monohydroxyalkyl- oder $C_{3-6}$-Polyhydroxyalkylrest bedeutet, in einem kosmetisch zulässigen Trägermedium und zusätzlich mindestens einen kosmetischen Hilfstoff enthält, ausgewählt aus anionischen, kationischen, nicht-ionischen, amphoteren oberflächenaktiven Mitteln oder deren Mischungen, Verdickungs-, Dispergier-, Eindring-, Sequestrierungs-, filmbildenden Mitteln, Puffern, Parfüms, alkalisch oder sauer machenden Mitteln, opak machenden Mitteln, Haarbehandlungsmitteln oder Konservierungsstoffen.

**4.** Kosmetische Zusammensetzung gemäß Anspruch 3,
dadurch **gekennzeichnet,** daß
R $-CH_2-CHOH-CH_2OH$ oder $-CH_2-CH_2OH$ darstellt.

**5.** Färbezusammensetzung für keratinische Fasern,
dadurch **gekennzeichnet,** daß
sie mindestens ein Anthrachinon der Formel (I) :

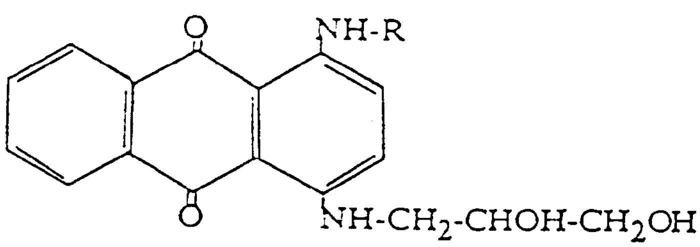

(I)

worin R einen $C_{2-6}$-Monohydroxyalkyl- oder $C_{3-6}$-Polyhydroxyalkylrest darstellt, zusammen mit einem oder mehreren weiteren Farbstoffen, ausgewählt aus:
(i) nitrierten Farbstoffen des benzolischen Typs;
(ii) Farbstoffen des Azotyps;
(iii) Oxidationsfarbstoffvorstufen,
in einem kosmetisch zulässigen Trägermedium umfaßt.

**6.** Zusammensetzung nach Anspruch 5,
dadurch **gekennzeichnet,** daß
das Anthrachinon der Formel (I) eines ist, worin R $-CH_2-CHOH-CH_2OH$ oder $CH_2-CH_2OH$ darstellt.

**7.** Zusammensetzung gemäß Anspruch 5 oder 6,
dadurch **gekennzeichnet,** daß
die nitrierten Farbstoffe des benzolischen Typs aus blauen oder violetten Farbstoffen des Typs Nitro-p-phenylendiamin ausgewählt sind, welche einen Farbton gemäß Munsell von 2,5 B bis 10 RP aufweisen.

**8.** Zusammensetzung gemäß Anspruch 7,
dadurch **gekennzeichnet,** daß
die benzolischen Farbstoffe des nitrierten Typs ausgewählt sind aus:
- 2-N-Methylamino-5-(N,N-bis(β-hydroxyethyl)amino)nitrobenzol;
- 2-N-Methylamino-5-(N-Methyl,N-(β-hydroxyethyl)amino)nitrobenzol;
- 2-(N-β-Hydroxyethyl)amino-5-(N,N-bis(β-hydroxyethyl)amino)nitrobenzol;

- 2-(N-β-Hydroxyethyl)amino-5-(N-methyl,N-(β-hydroxyethyl)amino)nitrobenzol;
- 2-(N-γ-Hydroxypropylamino)-5-(N,N-bis(β-hydroxyethyl)amino)nitrobenzol;
- 2-(N-β-Aminoethylamino)-5-(N,N-bis(β-hydroxyethyl)amino)nitrobenzol;
- 2-N-Methylamino-5-(N-methyl,N-(β,γ-dihydroxypropyl)amino)nitrobenzol;
- 2-Amino-5-(N,N-bis(β-hydroxyethyl)amino)nitrobenzol;
- 2-N-(β-Hydroxyethyl)amino-5-(N-(β-hydroxyethyl)amino)nitrobenzol;
- 2-N-(β-Hydroxyethylamino-5-(N-ethyl,N-(β-hydroxyethyl)aminonitrobenzol.

**9.** Zusammensetzung gemäß jedem Anspruch 5 bis 8,
dadurch **gekennzeichnet,** daß
die nitrierten Farbstoffe des benzolischen Typs aus gelben, orangefarbenen oder roten Nitrophenylendiaminen, Nitroaminophenolen, Nitroaminoalkoxybenzolen oder Nitroaminohhydroxyalkoxybenzolen ausgewählt sind.

**10.** Zusammensetzung gemäß Anspruch 9,
dadurch **gekennzeichnet,** daß
die nitrierten Farbstoffe des benzolischen Typs ausgewählt sind aus:

- 2-Amino-5-N-methylaminonitrobenzol,
- 2,4-Diaminonitrobenzol,
- 3,4-Diaminonitrobenzool,
- 2,5-Diaminonitrobenzol,
- 3-Amino-4-hydroxynitrobenzol,
- 3-Hydroxy-4-aminonitrobenzol,
- 2-Hydroxy-5-aminonitrobenzol,
- 2-Amino-5-hydroxynitrobenzol,
- 2-Amino-3-hydroxynitrobenzol,
- 2-Amino-5-N-(β-hydroxyethyl)aminonitrobenzol,
- 2-N-(β-Hydroxyethyl)amino-5-hydroxynitrobenzol,
- 3-Methoxy-4-N-(β-hydroxyethyl)aminonitrobenzol,
- 2-N-Methylamino-4-(β-hydroxyethyloxy)nitrobenzol,
- 2-Amino-3-methylnitrobenzol,
- 2-N-(β-Hydroxyethyl)amino-5-aminonitrobenzol,
- 2-Amino-4-chlor-5-N-(β-hydroxyethyl)aminonitrobenzol,
- 2-Amino-4-methyl-5-N-(β-hydroxyethyl)aminonitrobenzol,
- 2-Amino-4-methyl-5-N-methylaminonitrobenzol,
- 2-N-(β-Hydroxyethyl)amino-5-methoxynitrobenzol,
- 2-Amino-5(β-hydroxyethyloxy)nitrobenzol,
- 2-N-(β-Hydroxyethyl)aminonitrobenzol,
- 3-Amino-4-N-(β-hydroxyethyl)aminonitrobenzol,
- 3-(β-Hydroxyethyloxy)-4-N-(β-hydroxyethyl)aminonitrobenzol,
- 2-N-Methylamino-4-(β,γ-dihydroxypropyloxy)nitrobenzol,
- 2-N- (β-Hydroxyethyl)amino-5-(β-hydroxyethyloxy)nitrobenzol,
- 2-N-(β-Hydroxyethyl)amino-5-(β,γ-dihydroxypropyloxy)nitrobenzol,
- 3-Hydroxy-4-N-(β-hydroxyethyl)aminonitrobenzol,
- 3-N- (β-Hydroxyethyl)amino-4-N-(β-hydroxyethyl)aminonitrobenzol,
- 2-Amino-4-methyl-5-N-(β,γ-dihydroxypropyl)aminonitrobenzol,
- 2-Amino-4-methyl-5-hydroxynitrobenzol,
- 2-N-(β-Aminoethyl)amino-4-methoxynitrobenzol,
- 2-N-(β-Aminoethyl)aminonitrobenzol,
- 2-N-(β-Aminoethyl)amino-5-N-(β-hydroxyethyl)aminonitrobenzol,
- 2-Amino-4-methyl-5N-(β-aminoethyl)aminonitrobenzol,
- 2-Amino-4-chlor-5N-(β-aminoethyl)aminonitrobenzol,
- 2-N- (β-Hydroxypropyl)amino-5-hydroxynitrobenzol,
- 2,6-Diaminonitrobenzol,
- 2-N-(β-Hydroxyethyl)amino-6-aminonitrobenzol,
- 2,6-Di(β-hydroxyethylamino)nitrobenzol,
- 2-N-Ethylamino-5-carboxynitrobenzol,
- 2-Nitro-4-aminodiphenylamin-2'-carboxylsäure,

- 3-N-Ethylamino-4-hydroxy-5-chlornitrobenzol,
- 2-N-(β-Hydroxyethyl)amino-5-methoxy-2,3-dinitrobenzol,
- 2-N-(β,γ-Dihydroxypropyl)amino-5-methoxy-2,3-dinitrobenzol,
- 2-N-(β-Hydroxyethyl)amino-5-trifluormethylnitrobenzol,
- 2-N-(β,γ-Dihyroxypropyl)amino-5-trifluormethylnitrobenzol,
- 2-N-(β-Hydroxyethyl)amino-5-methylnitrobenzol.

**11.** Zusammensetzung gemäß jedem Anspruch 5 bis 10,
dadurch **gekennzeichnet,** daß
sie zusätzlich nitrierte Farbstoffe des Nitropyridin-Typs oder weitere Anthrachinonverbindungen umfaßt.

**12.** Zusammensetzung gemäß jedem Anspruch 5 bis 11,
dadurch **gekennzeichnet,** daß
der Azofarbstoff ausgewählt ist aus:
- 1-(4'-Aminophenylazo)-4-nitrobnezol,
- 1-(4'-Nitrophenylazo)-2-methyl-4-di(β-hydroxyethyl)aminobenzol,
- 1-(4'-Aminodiphenylazo)-2-methyl-4-di(β-hydroxyethyl)aminobenzol,
- 1-(4'-Di(β-hydroxyethyl) aminophenylazo)-4-aminobenzol oder
- 4-Amino-2'-methyl-4'-N,N-di (β-hydroxyethyl) aminophenylazobenzol.

**13.** Zusammensetzung gemäß jedem Anspruch 5 bis 12,
dadurch **gekennzeichnet,** daß
die Oxidationsfarbstoffvorstufen aus p-Phenylendiaminen, p-Aminophenolen, heterozyklischen Basen, o-Aminophenolen ausgewählt sind.

**14.** Zusammensetzung gemäß jedem Anspruch 5 bis 13,
dadurch **gekennzeichnet,** daß
sie Kuppler umfaßt, ausgewählt aus m-Diphenolen, m-Aminophenolen, m-Phenylendiaminen, m-Acylaminophenolen, m-Ureidophenolen, m-Carbalkoxiaminophenolen, α-Naphthol, Diacetonverbindungen und heterozyklischen Kupplern.

**15.** Zusammensetzung gemäß jedem Anspruch 5 bis 14,
dadurch **gekennzeichnet,** daß
sie 0,01 bis 5 Gew.% Anthrachinonfarbstoff(e) enthält.

**16.** Zusammensetzung gemäß jedem Anspruch 5 bis 15,
dadurch **gekennzeichnet,** daß
sie 0,01 bis 10 Gew.% nitrierte(n) Farbstoff(e) des benzolischen Typs enthält.

**17.** Zusammensetzung gemäß jedem Anspruch 5 bis 16,
dadurch **gekennzeichnet,** daß
sie 0,005 bis 5 Gew.% Azofarbstoff(e) enthält.

**18.** Zusammensetzung gemäß jedem Anspruch 5 bis 17,
dadurch **gekennzeichnet,** daß
sie 0,01 bis 10 Gew.% und vorzugsweise 0,03 bis 5 Gew.% Oxidationsfarbstoffvorstufen enthält.

**19.** Zusammensetzung gemäß jedem Anspruch 5 bis 18,
dadurch **gekennzeichnet,** daß
sie 0,001 bis 8 Gew.% und vorzugsweise 0,015 bis 5 Gew.% Kuppler enthält.

**20.** Zusammensetzung gemäß jedem Anspruch 5 und 13 bis 19,
dadurch **gekennzeichnet,** daß
sie zusätzlich Reduktions- oder antioxidierende Mittel in Mengenverhältnissen von 0,05 bis 3 Gew.% des Gesamtgewichts der Zusammensetzung enthält.

**21.** Zusammensetzung gemäß jedem Anspruch 5 bis 20,
dadurch **gekennzeichnet,** daß
sie zusätzlich kosmetische Hilfstoffe umfaßt, ausgewählt aus anionischen, kationischen, nicht-ionischen,

amphoteren oberflächenaktiven Mitteln oder deren Mischungen, Verdickungs-, Dispergier-, Eindring-, Sequestrierungs-, filmbildenden Mitteln, Puffern, Parfüms, alkalisch oder sauer machenden Mitteln, Perlmuttglanz ergebenden Mitteln oder Solubilisierungsmitteln.

**22.** Direktfärbeverfahren von keratinischen Fasern,
dadurch **gekennzeichnet,** daß
man auf die keratinischen Fasern eine gemäß einem der Ansprüche 3 bis 12, 15 bis 17 und 21 definierte Zusammensetzung aufträgt.

**23.** Oxidationsfärbeverfahren von keratinischen Fasern,
dadurch **gekennzeichnet,** daß
man auf die keratinischen Fasern eine Zusammensetzung gemäß einem der Ansprüche 3 bis 21 aufträgt, wobei die Aufbringung dieser Zusammensetzung zusammen mit oder im Anschluß an die Aufbringung einer Zusammensetzung erfolgt, die ein oxidierendes Mittel in einem kosmetisch zulässigen Medium enthält.

**24.** Kit zur Färbung keratinischer Fasern,
dadurch **gekennzeichnet,** daß
es mindestens einen Teil, der eine Zusammensetzung gemäß einem der Ansprüche 3 bis 21 enthält, und mindestens einen Teil umfaßt, der ein Oxidationsmittel in einem kosmetisch zulässigen Lösungsmittel enthält.

**Patentansprüche für folgenden Vertragsstaat : ES**

**1.** Verwendung von Anthrachinon der Formel (I) :

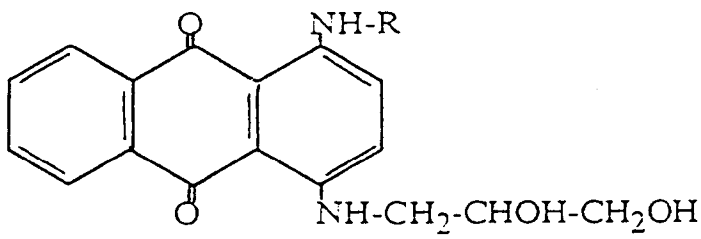

(I)

worin R einen $C_{2-6}$-Monohydroxyalkyl- oder $C_{3-6}$-Polyhydroxyalkylrest bedeutet, zur Färbung keratinischer menschlicher Fasern.

**2.** Verwendung gemäß Anspruch 1,
dadurch **gekennzeichnet,** daß
das Anthrachinon 1,4-Di(β,γ-dihydroxypropylamino)-9,10-anthrachinon oder 1-(β-Hydroxyethylamino)-4-(β,γ-dihydroxypropylamino)-9,10-anthrachinon ist.

**3.** Kosmetische Zusammensetzung,
dadurch **gekennzeichnet,** daß
sie ein Anthrachinon der Formel (I) :

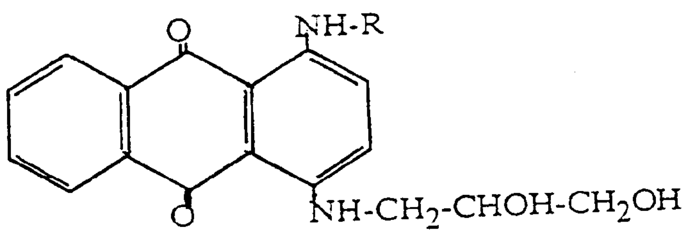

(I)

worin R einen $C_2$-$C_6$-Monohydroxyalkyl- oder $C_{3-6}$-Polyhydroxyalkylrest bedeutet, in einem kosmetisch zulässigen Trägermedium und zusätzlich mindestens einen kosmetischen Hilfstoff enthält, ausgewählt

aus anionischen, kationischen, nicht-ionischen, amphoteren oberflächenaktiven Mitteln oder deren Mischungen, Verdickungs-, Dispergier-, Eindring-, Sequestrierungs-, filmbildenden Mitteln, Puffern, Parfüms, alkalisch oder sauer machenden Mitteln, opak machenden Mitteln, Haarbehandlungsmitteln oder Konservierungsstoffen.

4. Kosmetische Zusammensetzung gemäß Anspruch 3,
dadurch **gekennzeichnet,** daß
R $-CH_2-CHOH-CH_2OH$ oder $-CH_2-CH_2OH$ darstellt.

5. Färbezusammensetzung für keratinische Fasern,
dadurch **gekennzeichnet,** daß
sie mindestens ein Anthrachinon der Formel (I)

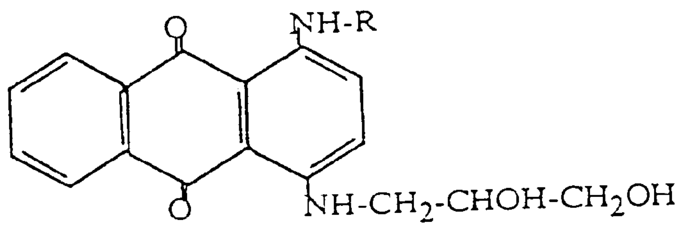

(I)

worin R einen $C_{2-6}$-Monohydroxyalkyl- oder $C_{3-6}$-Polyhydroxyalkylrest darstellt, zusammen mit einem oder mehreren weiteren Farbstoffen, ausgewählt aus:
(i) nitrierten Farbstoffen des benzolischen Typs;
(ii) Farbstoffen des Azotyps;
(iii) Oxidationsfarbstoffvorstufen, in einem kosmetisch zulässigen Trägermedium, umfaßt.

6. Zusammensetzung nach Anspruch 5,
dadurch **gekennzeichnet,** daß
das Anthrachinon der Formel (I) eines ist, worin R $-CH_2-CHOH-CH_2OH$ oder $CH_2-CH_2OH$ darstellt.

7. Zusammensetzung gemäß Anspruch 5 oder 6,
dadurch **gekennzeichnet,** daß
die nitrierten Farbstoffe des benzolischen Typs aus blauen oder violetten Farbstoffen des Typs Nitro-p-phenylendiamin ausgewählt sind, welche einen Farbton gemäß Munsell von 2,5 B bis 10 RP aufweisen.

8. Zusammensetzung gemäß Anspruch 7,
dadurch **gekennzeichnet,** daß
die benzolischen Farbstoffe des nitrierten Typs ausgewählt sind aus:
- 2-N-Methylamino-5-(N,N-bis(β-hydroxyethyl)amino)nitrobenzol;
- 2-N-Methylamino-5-(N-Methyl,N-(β-hydroxyethyl)amino)nitrobenzol;
- 2-(N-β-Hydroxyethyl)amino-5-(N,N-bis(β-hydroxyethyl)amino)nitrobenzol;
- 2-(N-β-Hydroxyethyl)amino-5-(N-methyl,N-(β-(hydroxyethyl)amino)-nitro-benzol;
- 2-(N-γ-Hydroxypropylamino)-5-(N,N-bis(β-hydroxyethyl)amino)nitrobenzol;
- 2-(N-β-Aminoethylamino)-5-(N,N-bis(β-hydroxyethyl)amino)nitrobenzol;
- 2-N-Methylamino-5-(N-methyl,N-(β.γ-dihydroxypropyl)amino)-nitrobenzol;
- 2-Amino-5-(N,N-bis(β-hydroxyethyl)amino)nitrobenzol;
- 2-N-(β-Hydroxyethyl)amino-5-(N-(β-hydroxyethyl)amino)nitrobenzol;
- 2-N-(β-Hydroxyethylamino-5-(N-ethyl,N-(β-hydroxyethyl)aminonitrobenzol.

9. Zusammensetzung gemäß jedem Anspruch 5 bis 8,
dadurch **gekennzeichnet,** daß
die nitrierten Farbstoffe des benzolischen Typs aus gelben, orangefarbenen oder roten Nitrophenylendiaminen, Nitroaminophenolen, Nitroaminoalkoxybenzolen oder Nitroaminohydroxyalkoxybenzolen ausgewählt sind.

10. Zusammensetzung gemäß Anspruch 9,
dadurch **gekennzeichnet,** daß

die nitrierten Farbstoffe des benzolischen Typs ausgewählt sind aus:
- 2-Amino-5-N-methylaminonitrobenzol,
- 2,4-Diaminonitrobenzol,
- 3,4-Diaminonitrobenzool,
- 2,5-Diaminonitrobenzol,
- 3-Amino-4-hydroxynitrobenzol,
- 3-Hydroxy-4-aminonitrobenzol,
- 2-Hydroxy-5-aminonitrobenzol,
- 2-Amino-5-hydroxynitrobenzol,
- 2-Amino-3-hydroxynitrobenzol,
- 2-Amino-5-N- (β-hydroxyethyl)aminonitrobenzol,
- 2-N-(β-Hydroxyethyl)amino-5-hydroxynitrobenzol,
- 3-Methoxy-4-N-(β-hydroxyethyl)aminonitrobenzol,
- 2-N-Methylamino-4-(β-hydroxyethyloxy)nitrobenzol,
- 2-Amino-3-methylnitrobenzol,
- 2-N-(β-Hydroxyethyl)amino-5-aminonitrobenzol,
- 2-Amino-4-chlor-5-N-(β-hydroxyethyl)aminonitrobenzol,
- 2-Amino-4-methyl-5-N-(β-hydroxyethyl)aminonitrobenzol,
- 2-Amino-4-methyl-5-N-methylaminonitrobenzol,
- 2-N-(β-Hydroxyethyl)amino-5-methoxynitrobenzol,
- 2-Amino-5-(β-hydroxyethyloxy)nitrobenzol,
- 2-N-(β-Hydroxyethyl)aminonitrobenzol,
- 3-Amino-4-N-(β-hydroxyethyl)aminonitrobenzol,
- 3-(β-Hydroxyethyloxy)-4-N-(β-hydroxyethyl)aminonitrobenzol,
- 2-N-Methylamino-4-(β,γ-dihydroxypropyloxy)nitrobenzol,
- 2-N-(β-Hydroxyethyl)amino-5-(β-hydroxyethyloxy)nitrobenzol,
- 2-N-(β-Hydroxyethyl)amino-5-(β,γ-dihydroxypropyloxy)nitrobenzol,
- 3-Hydroxy-4-N-(β-hydroxyethyl)aminonitrobenzol,
- 3-N-(β-Hydroxyethyl)amino-4-N-(β-hydroxyethyl)aminonitrobenzol,
- 2-Amino-4-methyl-5-N- (β,γ-dihydroxypropyl)aminonitrobenzol,
- 2-Amino-4-methyl-5-hydroxynitrobenzol,
- 2-N-(β-Aminoethyl)amino-4-methoxynitrobenzol,
- 2-N-(β-Aminoethyl)aminonitrobenzol,
- 2-N-(β-Aminoethyl)amino-5-N-(β-hydroxyethyl)aminonitrobenzol,
- 2-Amino-4-methyl-5-N-(β-aminoethyl)aminonitrobenzol,
- 2-Amino-4-Cclor-5-N-(β-aminoethyl)aminonitrobenzol,
- 2-N-(β-Hydroxypropyl)amino-5-hydroxynitrobenzol,
- 2,6-Diaminonitrobenzol,
- 2-N-(β-Hydroxyethyl)amino-6-aminonitrobenzol,
- 2,6-Di(β-hydroxyethylamino)nitrobenzol,
- 2-N-Ethylamino-5-carboxynitrobenzol,
- 2-Nitro-4-aminodiphenylamin-2'-carboxylsäure,
- 3-N-Ethylamino-4-hydroxy-5-chlornitrobenzol,
- 2-N-(β-Hydroxyethyl)amino-5-methoxy-2,3-dinitrobenzol,
- 2-N-(β,γ-Dihydroxypropyl)amino-5-methoxy-2,3-dinitrobenzol,
- 2-N-(β-Hydroxyethyl)amino-5-trifluormethylnitrobenzol,
- 2-N-(β,γ-Dihyroxypropyl)amino-5-trifluormethylnitrobenzol,
- 2-N-(β-Hydroxyethyl)amino-5-methylnitrobenzol.

**11.** Zusammensetzung gemäß jedem Anspruch 5 bis 10, dadurch **gekennzeichnet,** daß sie zusätzlich nitrierte Farbstoffe des Nitropyridin-Typs oder weitere Anthrachinonverbindungen umfaßt.

**12.** Zusammensetzung gemäß jedem Anspruch 5 bis 11, dadurch **gekennzeichnet,** daß der Azofarbstoff ausgewählt ist aus:
- 1-(4'-Aminophenylazo)-4-nitrobnezol,
- 1-(4'-Nitrophenylazo)-2-methyl-4-di(β-hydroxyethyl)aminobenzol,

- 1-(4'-Aminodiphenylazo)-2-methyl-4-di(β-hydroxyethyl)aminobenzol,
- 1-(4'-Di(β-hydroxyethyl)aminophenylazo)-4-aminobenzol oder
- 4-Amino-2' -methyl-4' -N,N-di (β-hydroxyethyl)aminophenylazobenzol.

**13.** Zusammensetzung gemäß jedem Anspruch 5 bis 12,
dadurch **gekennzeichnet,** daß
die Oxidationsfarbstoffvorstufen aus p-Phenylendiaminen, p-Aminophenolen, heterozyklischen Basen, o-Aminophenolen ausgewählt sind.

**14.** Zusammensetzung gemäß jedem Anspruch 5 bis 13,
dadurch **gekennzeichnet,** daß
sie Kuppler umfaßt, ausgewählt aus m-Diphenolen, m-Aminophenolen, m-Phenylendiaminen, m-Acylaminophenolen, m-Ureidophenolen, m-Carbalkoxiaminophenolen, α-Naphthol, Diacetonverbindungen und heterozyklischen Kupplern.

**15.** Zusammensetzung gemäß jedem Anspruch 5 bis 14,
dadurch **gekennzeichnet,** daß
sie 0,01 bis 5 Gew.% Anthrachinonfarbstoff(e) enthält.

**16.** Zusammensetzung gemäß jedem Anspruch 5 bis 15,
dadurch **gekennzeichnet,** daß
sie 0,01 bis 10 Gew.% nitrierte(n) Farbstoff(e) des benzolischen Typs enthält.

**17.** Zusammensetzung gemäß jedem Anspruch 5 bis 16,
dadurch **gekennzeichnet,** daß
sie 0,005 bis 5 Gew.% Azofarbstoff(e) enthält.

**18.** Zusammensetzung gemäß jedem Anspruch 5 bis 17,
dadurch **gekennzeichnet,** daß
sie 0,01 bis 10 Gew.% und vorzugsweise 0,03 bis 5 Gew.% Oxidationsfarbstoffvorstufen enthält.

**19.** Zusammensetzung gemäß jedem Anspruch 5 bis 18,
dadurch **gekennzeichnet,** daß
sie 0,001 bis 8 Gew.% und vorzugsweise 0,015 bis 5 Gew.% Kuppler enthält.

**20.** Zusammensetzung gemäß jedem Anspruch 5 und 13 bis 19,
dadurch **gekennzeichnet,** daß
sie zusätzlich Reduktions- oder antioxidierende Mittel in Mengenverhältnissen von 0,05 bis 3 Gew.% des Gesamtgewichts der Zusammensetzung enthält.

**21.** Zusammensetzung gemäß jedem Anspruch 5 bis 20,
dadurch **gekennzeichnet,** daß
sie zusätzlich kosmetische Hilfstoffe umfaßt, ausgewählt aus anionischen, kationischen, nicht-ionischen, amphoteren oberflächenaktiven Mitteln oder deren Mischungen, Verdickungs-, Dispergier-, Eindring-, Sequestrierungs-, filmbildenden Mitteln, Puffern, Parfüms, alkalisch oder sauer machenden Mitteln, Perlmuttglanz ergebenden Mitteln oder Solubilisierungsmitteln.

**22.** Direktfärbeverfahren von keratinischen Fasern,
dadurch **gekennzeichnet,** daß
man auf die keratinischen Fasern eine gemäß einem der Ansprüche 3 bis 12, 15 bis 17 und 21 definierte Zusammensetzung aufträgt.

**23.** Oxidationsfärbeverfahren von keratinischen Fasern,
dadurch **gekennzeichnet,** daß
man auf die keratinischen Fasern eine Zusammensetzung gemäß einem der Ansprüche 3 bis 21 aufträgt, wobei die Aufbringung dieser Zusammensetzung zusammen mit oder im Anschluß an die Aufbringung einer Zusammensetzung erfolgt, die ein oxidierendes Mittel in einem kosmetisch zulässigen Medium enthält.

**24.** Kit zur Färbung keratinischer Fasern,

dadurch **gekennzeichnet,** daß
es mindestens einen Teil, der eine Zusammensetzung gemäß einem der Ansprüche 3 bis 21 enthält, und mindestens einen Teil umfaßt, der ein Oxidationsmittel in einem kosmetisch zulässigen Lösungsmittel enthält.

**25.** Verfahren zur Herstellung einer kosmetischen Zusammensetzung,
dadurch **gekennzeichnet,** daß
man mindestens ein Anthrachinon der Formel (I) :

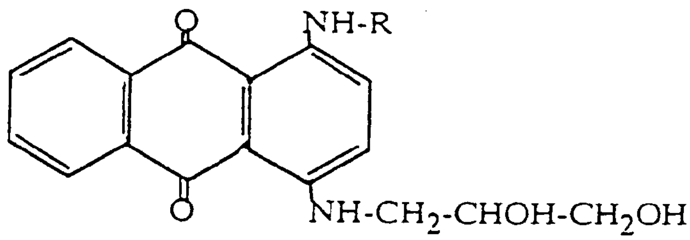

(I)

worin R einen $C_{2-6}$-Monohydroxyalkyl- oder $C_{3-6}$-Polyhydroxyalkylrest darstellt, in einer Konzentration von 0,1 bis 5 Gew.%, bezogen auf Gesamtgewicht der Zusammensetzung, in ein kosmetisch zulässiges Trägermedium einbringt, das zusätzlich midnestens einen kosmetischen Hilfstoff enthält, ausgewählt aus anionischen, kationischen, nicht-ionischen, amphoteren oberflächenaktiven Mitteln oder deren Mischungen, Verdickungs-, Dispergier-, Eindring-, Sequestrierungs-, filmbildenden Mitteln, Puffern, Parfüms, alkalisch oder sauer machenden Mitteln, opak machenden Mitteln, Haarbehandlungsmitteln oder Konservierungsstoffen.

## Claims

### Claims for the following Contracting States : AT, BE, CH, DE, DK, GB, GR, IT, LI, NL, SE

**1.** Use of anthraquinones of formula (I):

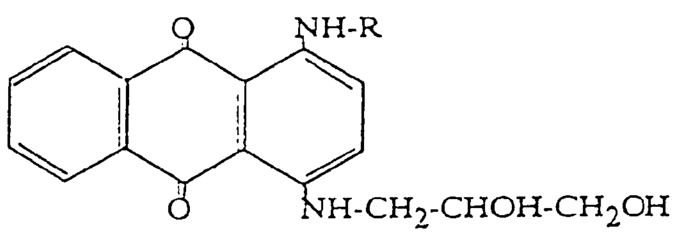

(I)

where R represents a $C_2$ to $C_6$ monohydroxyalkyl radical or a $C_3$ to $C_6$ polyhydroxyalkyl radical, for dyeing human keratinous fibres.

**2.** Use according to Claim 1, characterised in that the anthraquinone is 1,4-di-(β,γ-dihydroxypropylamino) 9,10-anthraquinone or 1-(β-hydroxyethylamino)-4-(β,γ-dihydroxypropylamino)-9,10-anthraquinone.

**3.** Cosmetic composition, characterised in that it comprises an anthraquinon of formula (I):

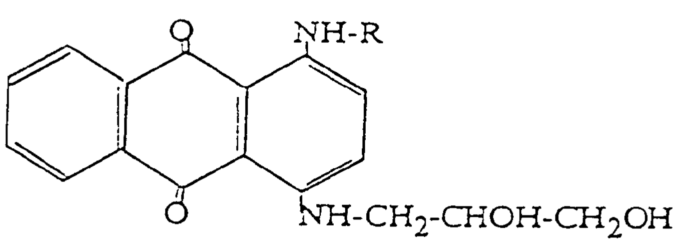

(I)

where R represents a $C_2$-$C_5$ monohydroxyalkyl radical or a $C_3$-$C_6$ polyhydroxyalkyl radical in a cosmetically acceptable carrier, and in addition, at least one of the cosmetic adjuvants chosen from anionic, cationic, non-ionic or amphoteric surface-active agents or their mixtures, thickeners, dispersing agents, penetrating agents, sequestrants, film-forming agents, buffers, perfumes, alkalinising or acidifying agents, opacifying agents, hair treating agents or preservatives.

**4.** Cosmetic composition according to Claim 3, characterised in that R represents $CH_2$-CHOH-$CH_2$OH or $CH_2$-$CH_2$OH .

**5.** Dyeing composition for keratinous fibres, characterised in that it comprises at least one anthraquinone of formula (I):

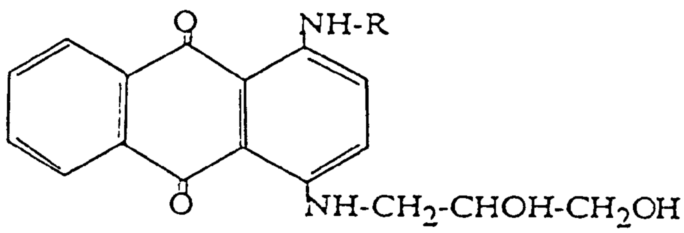

(I)

where R represents a $C_2$-$C_6$ monohydroxyalkyl or a $C_3$-$C_6$ polyhydroxyalkyl radical, in combination with one or more other dyes chosen from:
(i) nitro dyes of the benzene type;
(ii) dyes of the azo type;
(iii) oxidation dye precursors,
in a cosmetically acceptable carrier.

**6.** Composition according to Claim 5, characterised in that the anthraquinone of formula (I) is that where R represents $CH_2$-CHOR-$CH_2$OH or $CH_2$-$CH_2$OH.

**7.** Composition according to Claim 5 or 6, charac- terised in that the nitro dyes of the benzene type are chosen from the blue or violet dyes of the nitroparaphenylenediamine type, having a shade according to Munsell of between 2.5 3 and 10 RP.

**8.** Composition according to Claim 7, characterised in that the benzene dyes of the nitro type are chosen from:
- 2-N-methylamino-5-[N,N-bis(β-hydroxyethyl)amino]nitrobenzene;
- 2-N-methylamino-5-[N,methyl-N-(β-hydroxyethyl)amino]nitrobenzene;
- 2-(N-β-hydroxyethyl)amino-5-[N-N-bis(β-hydroxyethyl)amino]nitrobenzene;
- 2-(N-β-hydroxyethyl)amino-5-[N-methyl-N-(β-hydroxyethyl)amino]nitrobenzene;
- 2-(N-γ-hydroxypropyl)amino-5-[N,N-bis(β-hydroxyethyl)amino]nitrobenzene;
- 2-(N-β-aminoethyl)amino-5-[N,N-bis(β-hydroxyethyl)amino]nitrobenzene;
- 2-N-methylamino-5-[N-methyl-N-(β,γ-dihydroxypropyl)amino]nitrobenzene;
- 2-amino-5-[N,N-bis(β-hydroxyethyl)amino]nitrobenzene;
- 2-N-(β-hyhroxyethyl)amino-5-[N-(β-hydroxyethyl)amino]nitrobenzene;
- 2-N-(β-hydroxyethyl)amino-5-[N-ethyl-N-(β-hydroxyethyl)amino]nitrobenzene.

**9.** Composition according to one of Claims 5 to 8, characterised in that the nitro dyes of the benzene type are chosen from the yellow, orange or red nitrophenylenediamines, the nitroaminophenols, the nitroaminoalkoxybenzenes, or the nitroaminohydroxyalkoxybenzenes

**10.** Composition according to Claim 9, characterised in that the nitro dyes of the benzene type are chosen from:
- 2-amino-5-N-methylaminonitrobenzene,
- 2,4-diaminonitrobenzene,
- 3,4-diaminonitrobenzene,
- 2,5-diaminonitrobenzene,
- 3-amino-4-hydroxynitrobenzene,

- 3-hydroxy-4-aminonitrobenzene,
- 2-hydroxy-5-aminonitrobenzene,
- 2-amino-5-hydroxynitrobenzene,
- 2-amino-3-hydroxynitrobenzene,
- 2-amino-5-N-(β-hydroxyethyl)aminonitrobenzene,
- 2-N-(β-hydroxyethyl)amino-5-hydroxynitrobenzene,
- 3-methoxy-4-N(β-hydroxyethyl)aminonitrobenzene,
- 2-N-methylanino-4(β-hydroxyethyloxy)nitrobenzene,
- 2-amino-3-methylnitrobenzene,
- 2-N-(β-hydroxyethyl)amino-5-aminonitrobenzene,
- 2-amino-4-chloro-5-N-(β-hydroxyethyl)aminonitrobenzene
- 2-amino-4-methyl-5-N-(β-hydroxyethyl)aminonitrobenzene,
- 2-amino-4-methyl-5-N-methylaminonitrobenzene,
- 2-N-(β-hydroxyethyl)amino-5-methoxynitrobenzene,
- 2-amino-5-(β-hydroxyethyloxy)nitrobenzene,
- 2-N-(β-hydroxyethyl)aminonitrobenzene,
- 3-amino-4-N-(β-hydroxyethyl)aminonitrobenzene,
- 3-(β-hydroxyethyloxy)-4-N-(β-hydroxyethyl)aminonitrobenzene,
- 2-N-methylamino-4-(β,γ-dihydroxypropyloxy)nitrobenzene,
- 2-N-(β-hydroxyethyl)amino-5-(β-hydroxyethyloxy)nitrobenzene,
- 2-N-(β-hydroxyethyl)amino-5-(β,α-dihydroxypropyloxy)nitrobenzene,
- 3-hydroxy-4-N-(β-hydroxyethyl)aminonitrobenzene,
- 3-N-(β-hydroxyethyl)amino-4-N-(β-hydroxyethyl)aminonitrobenzene,
- 2-amino-4-methyl-5-N-(β,γ-dihydroxypropyl)aminonitrobenzene,
- 2-amino-4-methyl-5-hydroxynitrobenzene,
- 2-N-(β-aminoethyl)amino-4-methoxynitrobenzene,
- 2-N-(β-aminoethyl)aminonitrobenzene,
- 2-N-(β-aminoethyl)amino-5-N-(β-hydroxyethyl)aminonitrobenzene,
- 2-amino-4-methyl-5-N-(β-aminoethyl)aminonitrobenzene,
- 2-amino-4-chloro-5-N-(β-aminoethyl)aminonitrobenzene,
- 2-N-(β-hydroxypropyl)amino-5-hydroxynitrobenzene,
- 2,6-diaminonitrobenzene,
- 2-N-(β-hydroxyethyl)amino-6-aminonitrobenzene,
- 2,6-di(β-hydroxyethylamino)nitrobenzene,
- 2-N-ethylamino-5-carboxynitrobenzene,
- 2-nitro-4-aminodiphenylamine-2'-carboxylic acid,
- 3-N-ethylamino-4-hydroxy-5-chloronitrobenzene,
- 2-N-(β-hydroxyethyl)amino-5-methoxy-2,3-dinitrobenzene,
- 2-N-(β,γ-dihydroxypropyl)amino-5-methoxy-2,3-dinitrobenzene,
- 2-N-(β-hydroxyethyl)amino-5-trifluoromethylnitrobenzene,
- 2-N-(β,γ-dihydroxypropyl)amino-5-trifluoromethylnitrobenzene,
- 2-N-(β-hydroxyethyl)amino-5-methylnitrobenze.

**11.** Composition according to one of Claims 5 to 10, characterised in that it comprises in addition nitro dyes of the nitropyridine type or other anthraquinone compounds.

**12.** Composition according to one of Claims 5 to 11, characterised in that the azo dye is chosen from:
- 1-(4'-aminophenylazo)-4-nitrobenzene,
- 1-(4'-nitrophenylazo)-2-methyl-4-di(β-hydroxyethyl)aminobenzene,
- 1'(4'-aminodiphenylazo)-2-methyl-4-di(β-hydroxyethyl)aminobenzene,
- 1-[4'-di(β-hydroxyethyl)aminophenylazo]-4-aminobenzene, or
- 4-amino-2'-methyl-4'-N,N-di(β-hydroxyethyl)aminophenylazobenzene.

**13.** Composition according to one of Claims 5 to 12, characterised in that the oxidation dye precursors are chosen from para-phenylenediamines, para-aminophenols, heterocyclic bases and ortho-aminophenols.

**14.** Composition according to one of Claims 5 to 13, characterised in that it comprises the couplers chosen from meta-diphenols, meta-aminophenols, meta-phenylenediamines, meta-acylaminophenols, meta-ureidophenols, meta-carbalkoxyaminophenols, alpha-naphthol, diketonic compounds, and heterocyclic couplers.

**15.** Composition according to one of Claims 5 to 14, characterised in that it comprises 0.01 to 5% by weight of anthraquinone dye(s).

**16.** Composition according to one of Claims 5 to 15, characterised in that it comprises between 0.01 and 10% by weight of nitro dye(s) of the benzene type.

**17.** Composition according to one of Claims 5 to 16, characterised in that it comprises between 0.005 and 5% by weight of azo dye(s).

**18.** Composition according to one of Claims 5 to 17, characterised in that it comprises between 0.01 and 10% by weight of oxidation dye precursors, preferably between 0.03 and 5% by weight.

**19.** Composition according to one of Claims 5 to 18, characterised in that it comprises between 0.001 and 8% by weight of couplers, preferably between 0.015 and 5% by weight.

**20.** Composition according to one of Claims 5 and 13 to 19, characterised in that it comprises in addition reducing or antioxidising agents in proportions of between 0.05 and 3% by weight of the total weight of the composition.

**21.** Composition according to one of Claims 5 to 20, characterised in that it comprises in addition cosmetic adjuvants chosen from anionic, cationic, nonionic, zmphoteric surface-active agents or their mixtures, thickeners, dispersing agents, penetrating agents, sequestrants, film-forming agents, buffers, perfumes, alkalinising or acidifying agents, pearling agents or solubilising agents.

**22.** Process for direct dyeing of keratinous fibres, characterised in that a composition such as that defined in one of Claims 3 to 12, 15 to 17 and 21 is applied to the keratinous fibres.

**23.** Process for oxidation dyeing of keratinous fibres, characterised in that a composition according to one of Claims 3 to 21, is applied to the keratinous fibres, the application of this composition being accompanied or followed by the application of a composition comprising an oxidising agent in a cosmetically acceptable medium.

**24.** Kit for dyeing keratinous fibres, characterised in that it comprises at least one compartment containing a composition according to one of Claims 3 to 21 and at least one compartment containing an oxidation agent in a cosmetically acceptable solvent.

**Claims for the following Contracting State : ES**

**1.** Use of anthraquinones of formula (I):

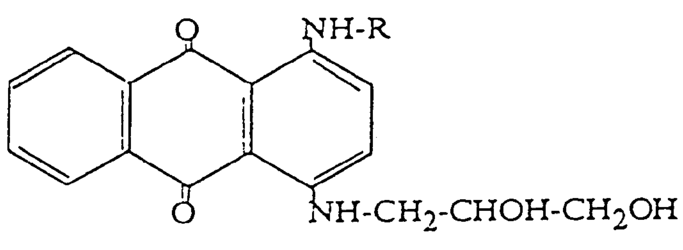

(I)

where R represents a $C_2$ to $C_6$ monohydroxyalkyl radical or a $C_3$ to $C_6$ polyhydroxyalkyl radical, for dyeing human keratinous fibres.

**2.** Use according to Claim 1, characterised in that the anthraquinone is 1,4-di-(β,γ-dihydroxypropylamino)9,10-anthraquinone or 1-(β-hydroxyethylamino)-4-(β,γ-dihydroxypropylamino)-9,10-anthraquinone.

**3.** Cosmetic composition, characterised in that it comprises an anthraquinone of formula (I):

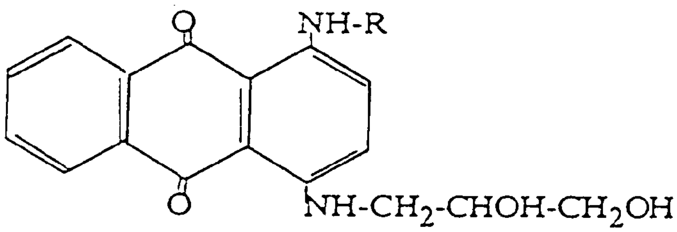

(I)

where R represents a $C_2$-$C_6$ monohydroxyalkyl radical or a $C_3$-$C_6$ polyhydroxyalkyl radical in a cosmetically acceptable carrier, and in addition, at least one of the cosmetic adjuvants chosen from anionic, cationic, non-ionic or amphoteric surface-active agents or their mixtures, thickeners, dispersing agents, penetrating agents, sequestrants, film-forming agents, buffers, perfumes, alkalinising or acidifying agents, opacifying agents, hair treating agents or preservatives.

**4.** Cosmetic composition according to Claim 3, characterised in that R represents $CH_2$-CHOH-$CH_2OH$ or $CH_2$-$CH_2OH$ .

**5.** Dyeing composition for keratinous fibres, characterised in that it comprises at least one anthraquinone of formula (I):

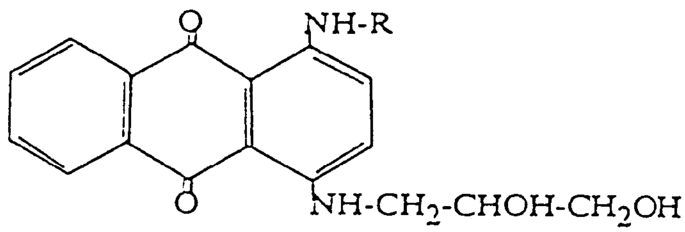

(I)

where R represents a $C_2$-$C_6$ monohydroxyalkyl or a $C_3$-$C_6$ polyhydroxyalkyl radical, in combination with one or more other dyes chosen from:

(i) nitro dyes of the benzene type;
(ii) dyes of the azo type;
(iii) oxidation dye precursors,

in a cosmetically acceptable carrier.

**6.** Composition according to Claim 5, characterised in that the anthraquinone of formula (I) is that where R represents $CH_2$-CHOH-$CH_2OH$ or $CH_2$-$CH_2OH$.

**7.** Composition according to Claim 5 or 6, charac- terised in that the nitro dyes of the benzene type are chosen from the blue or violet dyes of the nitroparaphenylenediamine type, having a shade according to Munsell of between 2.5 B and 10 RP.

**8.** Composition according to Claim 7, characterised in that the benzene dyes of the nitro type are chosen from:

- 2-N-methylamino-5-[N,N-bis(β-hydroxyethyl)amino]nitrobenzene;
- 2-N-methylamino-5-[N,methyl-N-(β-hydroxyethyl)amino]nitrobenzene;
- 2-(N-β-hydroxyethyl)amino-5-[N-N-bis(β-hydroxyethyl)amino]nitrobenzene;
- 2-(N-β-hydroxyethyl)amino-5-[N-methyl-N-(β-hydroxyethyl)amino]nitrobenzene;
- 2-(N-γ-hydroxypropyl)amino-5-[N,N-bis(β-hydroxyethyl)amino]nitrobenzene;
- 2-(N-β-aminoethyl)amino-5-[N,N-bis(β-hydroxyethyl)amino]nitrobenzene;
- 2-N-methylamino-5-[N-methyl-N-(β,γ-dihydroxypropyl)amino]nitrobenzene;
- 2-amino-5-[N,N-bis(β-hydroxyethyl)amino]nitrobenzene;
- 2-N-(β-hydroxyethyl)amino-5-[N-(β-hydroxyethyl)amino]nitrobenzene;
- 2-N-(β-hydroxyethyl)amino-5-[N-ethyl-N-(β-hydroxyethyl)amino]nitrobenzene.

**9.** Composition according to one of Claims 5 to 8, characterised in that the nitro dyes of the benzene type are chosen from the yellow, orange or red nitrophenylenediamines, the nitroaminophenols, the nitroaminoalkoxybenzenes, or the nitroaminohydroxyalkoxybenzenes.

**10.** Composition according to Claim 9, characterised in that the nitro dyes of the benzene type are chosen from:
- 2-amino-5-N-methylaminonitrobenzene,
- 2,4-diaminonitrobenzene,
- 3,4-diaminonitrobenzene,
- 2,5-diaminonitrobenzene,
- 3-amino-4-hydroxynitrobenzene,
- 3-hydroxy-4-aminonitrobenzene,
- 2-hydroxy-5-aminonitrobenzene,
- 2-amino-5-hydroxynitrobenzene,
- 2-amino-3-hydroxynitrobenzene,
- 2-amino-5-N-(β-hydroxyethyl)aminonitrobenzene,
- 2-N-(β-hydroxyethyl)amino-5-hydroxynitrobenzene,
- 3-methoxy-4-N-(β-hydroxyethyl)aminonitrobenzene,
- 2-N-methylamino-4-(β-hydroxyethyloxy)nitrobenzene,
- 2-amino-3-methylnitrobenzene,
- 2-N-(β-hydroxyethyl)amino-5-aminonitrobenzene,
- 2-amino-4-chloro-5-N-(β-hydroxyethyl)aminonitrobenzene,
- 2-amino-4-methyl-5-N-(β-hydroxyethyl)aminonitrobenzene,
- 2-amino-4-methyl-5-N-methylaminonitrobenzene,
- 2-N-(β-hydroxyethyl)amino-5-methoxynitrobenzene,
- 2-amino-5-(β-hydroxyethyloxy)nitrobenzene,
- 2-N-(β-hydroxyethyl)aminonitrobenzene,
- 3-amino-4-N-(β-hydroxyethyl)aminonitrobenzene,
- 3-(β-hydroxyethyloxy)-4-N-(β-hydroxyethyl)aminonitrobenzene,
- 2-N-methylamino-4-(β,γ-dihydroxypropyloxy)nitrobenzene,
- 2-N-(β-hydroxyethyl)amino-5-(β-hydroxyethyloxy)nitrobenzene,
- 2-N-(β-hydroxyethyl)amino-5-(β,α-dihydroxypropyloxy)nitrobenzene,
- 3-hydroxy-4-N-(β-hydroxyethyl)aminonitrobenzene,
- 3-N-(β-hydroxyethyl)amino-4-N-(β-hydroxyethyl)aminonitrobenzene,
- 2-amino-4-methyl-5-N-(β,γ-dihydroxypropyl)aminonitrobenzene,
- 2-amino-4-methyl-5-hydroxynitrobenzene,
- 2-N-(β-aminoethyl)amino-4-methoxynitrobenzene,
- 2-N-(β-aminoethyl)aminonitrobenzene,
- 2-N-(β-aminoethyl)amino-5-N-(β-hydroxyethyl)aminonitrobenzene,
- 2-amino-4-methyl-5-N-(β-aminoethyl)aminonitrobenzene,
- 2-amino-4-chloro-5-N-(β-aminoethyl)aminonitrobenzene,
- 2-N-(β-hydroxypropyl)amino-5-hydroxynitrobenzene,
- 2,6-diaminonitrobenzene,
- 2-N-(β-hydroxyethyl)amino-6-aminonitrobenzene,
- 2,6-di(β-hydroxyethylamino)nitrobenzene,
- 2-N-ethylamino-5-carboxynitrobenzene,
- 2-nitro-4-aminodiphenylamine-2'-carboxylic acid,
- 3-N-ethylamino-4-hydroxy-5-chloronitrobenzene,
- 2-N-(β-hydroxyethyl)amino-5-methoxy-2,3-dinitrobenzene,
- 2-N-(β,γ-dihydroxypropyl)amino-5-methoxy-2,3-dinitrobenzene,
- 2-N-(β-hydroxyethyl)amino-5-trifluoromethylnitrobenzene,
- 2-N-(β,γ-dihydroxypropyl)amino-5-trifluoromethylnitrobenzene,
- 2-N-(β-hydroxyethyl)amino-5-methylnitrobenzene.

**11.** Composition according to one of Claims 5 to 10, characterised in that it comprises in addition nitro dyes of the nitropyridine type or other anthraquinone compounds.

**12.** Composition according to one of Claims 5 to 11, characterised in that the azo dye is chosen from:
- 1-(4'-aminophenylazo)-4-nitrobenzene,
- 1-(4'-nitrophenylazo)-2-methyl-4-di(β-hydroxyethyl)aminobenzene,

- 1-(4'-aminodiphenylazo)-2-methyl-4'di(β-hydroxyethyl)aminobenzene,
- 1-[4'-di(β-hydroxyethyl)aminophenylazo]-4-aminobenzene, or
- 4-amino-2'-methyl-4'-N,N-di(β-hydroxyethyl)aminophenylazobenzene.

**13.** Composition according to one of Claims 5 to 12, characterised in that the oxidation dye precursors are chosen from para-phenylenediamines, para-aminophenols, heterocyclic bases and ortho-aminophenols.

**14.** Composition according to one of Claims 5 to 13, characterised in that it comprises the couplers chosen from meta-diphenols, meta-aminophenols, meta-phenylenediamines, meta-acylaminophenols, meta-ureidophenols, meta-carbalkoxyaminophenols, alpha-naphthol, diketonic compounds, and heterocyclic couplers.

**15.** Composition according to one of Claims 5 to 14, characterised in that it comprises 0.01 to 5% by weight of anthraquinone dye(s).

**16.** Composition according to one of Claims 5 to 15, characterised in that it comprises between 0.01 and 10% by weight of nitro dye(s) of the benzene type.

**17.** Composition according to one of Claims 5 to 16, characterised in that it comprises between 0.005 and 5% by weight of azo dye(s).

**18.** Composition according to one of Claims 5 to 17, characterised in that it comprises between 0.01 and 10% by weight of oxidation dye precursors, preferably between 0.03 and 5% by weight.

**19.** Composition according to one of Claims 5 to 18, characterised in that it comprises between 0.001 and 8% by weight of couplers, preferably between 0.015 and 5% by weight.

**20.** Composition according to one of Claims 5 and 13 to 19, characterised in that it comprises in addition reducing or antioxidising agents in proportions of between 0.05 and 3% by weight of the total weight of the composition.

**21.** Composition according to one of Claims 5 to 20, characterised in that it comprises in addition cosmetic adjuvants chosen from anionic, cationic, nonionic, amphoteric surface-active agents or their mixtures, thickeners, dispersing agents, penetrating agents, sequestrants, film-forming agents, buffers, perfumes, alkalinising or acidifying agents, pearling agents or solubilising agents.

**22.** Process for direct dyeing of keratinous fibres, characterised in that a composition such as that defined in one of Claims 3 to 12, 15 to 17 and 21 is applied to the keratinous fibres.

**23.** Process for oxidation dyeing of keratinous fibres, characterised in that a composition according to one of Claims 3 to 21, is applied to the keratinous fibres, the application of this composition being accompanied or followed by the application of a composition comprising an oxidising agent in a cosmetically acceptable medium.

**24.** Kit for dyeing keratinous fibres, characterised in that it comprises at least one compartment containing a composition according to one of Claims 3 to 21 and at least one compartment containing an oxidation agent in a cosmetically acceptable solvent.

**25.** Process for preparation of a cosmetic composition, characterised in that at least one anthraquinone of formula (I)

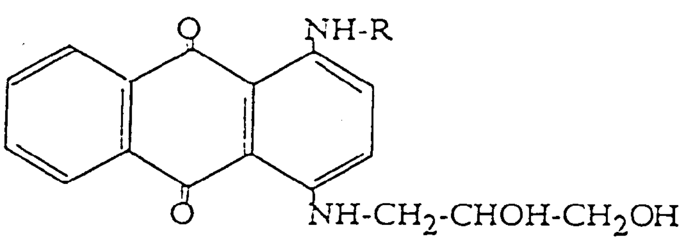

(I)

where R represents a $C_2$-$C_6$ monohydroxyalkyl radical or a $C_3$-$C_6$ polyhydroxyalkyl radical is incorporated at a concentration of 0.01 to 5 % by weight relative to the total weight of the composition, in a cosmetically acceptable carrier further containing at least one of the cosmetic adjuvants chosen from anionic, cationic,non-ionic or amphoteric surface-active agents or their mixtures, thickeners, dispersing agents, penetrating agents, sequestrants, film-forming agents, buffers, perfumes, alkalinising or acidifying agents, opacifying agents ,hair treating agents or preservatives.